# EUROPEAN PATENT SPECIFICATION

(11) **EP 4 424 709 B1**
(45) Date of publication and mention of the grant of the patent: **24.06.2026**
(21) Application number: 24162872.6
(22) Date of filing: 08.05.2020
(51) Int. Cl.: A61K 39/395, A61P 35/00, C07K 16/28, A61K 39/00

(54) **DOSING REGIMEN FOR ANTI-BCMA AGENTS**
DOSIERSCHEMA FÜR ANTI-BCMA-MITTEL
SCHÉMA POSOLOGIQUE POUR AGENTS ANTI-BCMA

(30) Priority: 11.11.2019 EP 19208417
(43) Date of publication of application: 04.09.2024
(62) Divisional of application: 20729951.2
(73) Proprietor: Amgen Research (Munich) GmbH, 81477 München (DE); Amgen Inc., Thousand Oaks, CA 91320-1799 (US)
(72) Inventor: STIEGLMAIER, Julia, 81477 Muenchen (DE); HUBER, Birgit, 81477 Muenchen (DE); MINELLA, Alexander, Thousand Oaks, 91320 (US); UPRETI, Vijay, Thousand Oaks, 91320 (US)
(74) Representative: Schiweck Weinzierl Koch Patentanwälte Partnerschaft mbB

(56) References cited:
- WO-A1-2017/134134
- WO-A1-2019/140196
- US-A1- 2013 273 055
- CHO S -F: "AMG 701, a half-life extended anti-BCMA BiTE , potently induces T cell-redirected lysis of human multiple myeloma cells and can be combined with IMiDs to overcome the immunosuppressive bone marrow microenvironment", CLINICAL LYMPHOMA, MYELOMA AND LEUKEMIA - 17TH INTERNATIONAL MYELOMA WORKSHOP 20191001 ELSEVIER INC. NLD, vol. 19, no. 10, Supplement, 1 October 2019 (2019-10-01), XP009519992, ISSN: 2152-2650
- YU-TZU TAI ET AL: "B cell maturation antigen (BCMA)-based immunotherapy for multiple myeloma", EXPERT OPINION ON BIOLOGICAL THERAPY, vol. 19, no. 11, 2 November 2019 (2019-11-02), ASHLEY, LONDON; GB, pages 1143 - 1156, XP055686682, ISSN: 1471-2598, DOI: 10.1080/14712598.2019.1641196
- EINSELE H ET AL: "OAB-025: The anti-BCMA Bispecific T-cell Engager (BiTE ) Molecule AMG 420 Induced MRD-Negative Complete Responses in R/R Multiple Myeloma in a FIH study", CLINICAL LYMPHOMA MYELOMA AND LEUKEMIA; 17TH INTERNATIONAL MYELOMA WORKSHOP, ELSEVIER, NL; BOSTON, MA, USA, vol. 19, no. 10, Supplement, 1 October 2019 (2019-10-01), pages e18 - e19, XP009519964, ISSN: 2152-2650, [retrieved on 20191018], DOI: 10.1016/J.CLML.2019.09.026
- TOPP M S ET AL: "8007: Evaluation of AMG 420, an anti-BCMA bispecific T-cell engager (BiTE) immunotherapy, in R/R multiple myeloma (MM) patients: Updated results of a first-in-human (FIH) phase i dose escalation study", JOURNAL OF CLINICAL ONCOLOGY; 2019 ANNUAL MEETING OF THE AMERICAN SOCIETY OF CLINICAL ONCOLOGY, ASCO 2019, LIPPINCOTT WILLIAMS & WILKINS, USA; CHICAGO, IL, USA, vol. 37, no. Supplement 15, 20 May 2019 (2019-05-20), pages 8007, XP009519965, ISSN: 1527-7755, [retrieved on 20190526], DOI: 10.1200/JCO.2019.37.15_SUPPL.8007
- TOPP MAX S ET AL: "Treatment with AMG 420, an Anti-B-CellMaturationAntigen (BCMA) Bispecific T-Cell Engager (BiTE (R)) Antibody Construct, Induces Minimal Residual Disease (MRD) Negative Complete Responses in Relapsed and/or Refractory (R/R) Multiple Myeloma (MM) Patients: Results of a First-in-Human (FIH) Phase I Dos", BLOOD; 60TH ANNUAL MEETING OF THE AMERICAN-SOCIETY-OF-HEMATOLOGY (ASH), THE AMERICAN SOCIETY OF HEMATOLOGY, US; SAN DIEGO, CA, USA, vol. 132, no. Suppl. 1, 29 November 2018 (2018-11-29), pages 1010, XP009519963, ISSN: 0006-4971, DOI: 10.1182/BLOOD-2018-99-109769
- TAI YU-TZU ET AL: "Novel anti-B-cell maturation antigen antibody-drug conjugate (GSK2857916) selectively induces killing of multiple myeloma", BLOOD, THE AMERICAN SOCIETY OF HEMATOLOGY, US, vol. 123, no. 20, 15 May 2014 (2014-05-15), pages 3128 - 3138, XP009505860, ISSN: 0006-4971, DOI: 10.1182/BLOOD-2013-10-535088
- ANONYMOUS CLINICALTRIALS: "Record History | ver. 20: 2019-10-23 | NCT03287908 | ClinicalTrials.gov", 23 October 2019 (2019-10-23), XP093289600, Retrieved from the Internet <URL:https://clinicaltrials.gov/study/NCT03287908?term=NCT03287908&rank=1&tab=history&a=20#version-content-panel>
- CHO SHIH-FENG ET AL: "Anti-BCMA BiTE (R) AMG701 Potently Induces Specific T Cell Lysis of Human Multiple Myeloma (MM) Cells and Immunomodulation in the Bone Marrow Microenvironment", BLOOD, ELSEVIER, AMSTERDAM, NL, vol. 132, no. Suppl. 1, 29 November 2018 (2018-11-29), pages 592, XP009519984, ISSN: 0006-4971, DOI: 10.1182/BLOOD-2018-99-118425
- CHO S-F ET AL: "AMG701 potently induces anti-multiple myeloma (MM) functions of T cells and IMiDs further enhance its efficacy to prevent MM relapse in vivo", BLOOD, W.B. SAUNDERS, vol. 134, no. Supplement 1, 1 November 2019 (2019-11-01), XP009519986, ISSN: 1528-0020
- LEDERMAN LYNNE: "Anti-BCMA BiTE AMG 701 Shows Preclinical Promise in Multiple Myeloma", ONCLIVE, 16 September 2019 (2019-09-16), pages 1 - 3, XP093241557, Retrieved from the Internet <URL:https://www.onclive.com/view/antibcma-bite-amg-701-shows-preclinical-promise-in-multiple-myeloma>
- DATABASE REGISTRY [online] 30 November 2018 (2018-11-30), ANONYMOUS: "CAS Registry: AM G-701", XP093241202, Database accession no. 2250292-39-6

## Description

### Field of the invention

The present invention relates to an antibody construct comprising a first domain which binds to BCMA, a second domain which binds to CD3 and a third domain which extends or enhances the half-life of the antibody construct, for use in the treatment or amelioration of a BCMA positive neoplasm, wherein the antibody construct is administered at a specified dose in at least one cycle as defined in the claims.

### Background of the invention

Multiple Myeloma (MM) is a malignant tumor of plasma cells which proliferate in bone marrow and release para protein. The resulting clinical laboratory pictures include infection, bone destruction, bone marrow failure, renal failure and hypercalcemia. The age adjusted annual incidence is increasing with approximately 6 new cases per 100,000. The incidence is 2 times higher in the black US population than in Caucasians. The 5-year survival rate for MM has increased from ~ 25% for newly diagnosed patients in 1975 to ~ 45% in 2006. This improvement is mainly due to new drugs such as proteasome inhibitors and immunomodulators. However, MM is not considered curable with current approaches. Patients refractory to proteasome inhibitors and immunomodulators show an unfavorable outcome with a median overall survival of 9 months.

Outcome is particularly poor in high-risk populations such as the subgroup with dell17p13 positive MM. Although many drugs are in clinical development for MM, new treatment options are still needed. Patients who show symptomatic disease are initially treated with primary induction therapy followed by high dose chemotherapy with autologous stem cell support in eligible patients. Patients eligible for intensive treatment are determined by age (65 to 75 years as upper limit), no comorbidities and intact renal function. Although this regimen has improved survival of younger and fit patients, the median duration of response does not exceed 3 years, and few patients remain free of the disease for more than 10 years.

Consolidation and maintenance approaches have been tested in order to increase the depth and duration of remission. Because maintenance treatment is challenging due to no efficacy or tolerability, there is still the option of improving survival outcome in the transplant setting by adding novel treatments to induction, consolidation or maintenance regimens. Patients not eligible for high dose therapy commonly receive induction regimens similar to the ones for the transplant candidates. These regimens include the proteasome inhibitor Bortezomib or a Melphalan based combination with Thalidomide. Median overall survival (OS) of Melphalan-Thalidomide-Prednisone (MPT) in elderly patients is 40 months. Lenalidomide in combination with Dexamethasone is the standard regimen for relapsed/refractory MM, but may move to the first line setting in transplant ineligible patients.

Other established regimens in the relapsed setting are repeat induction regimens or bortezomib or immunomodulator based salvage combinations with alkylating agents. An improvement of outcome (progression-free survival (PFS) and OS) is needed for patients in relapsed disease. Patients which are refractory to established treatments and progressing on treatment have a dismal outcome of 9 months OS on treatment and 3 months without treatment. The unmet need is highest in these patients.

Bispecific molecules such as BiTE^{®} (bispecific T cell engager) antibody constructs are recombinant protein constructs having (at least) one binding domain that is specific for a selected tumor-associated surface antigen on target cells, and a second binding domain that is specific for CD3, a subunit of the T cell receptor complex on T cells. By their particular design, BiTE^{®} antibody constructs are uniquely suited to transiently connect T cells with target cells and, at the same time, potently activate the inherent cytolytic potential of T cells against target cells. The first generation of so-called "canonical" (non-half-life-extended) BiTE^{®} antibody constructs (see WO 99/54440 and WO 2005/040220) was brought into the clinic as AMG 103 (blinatumomab, anti-CD19 x anti-CD3) and AMG 110 (solitomab, anti-EpCAM x anti-CD3). A further development of the first generation of canonical BiTE^{®} antibody constructs was the provision of bispecific antibody constructs binding to a context independent epitope at the N-terminus of the CD3-epsilon chain of human and *Callithrix jacchus, Saguinus oedipus* or *Saimiri sciureus* (WO 2008/119567). The first BiTE^{®} molecule comprising this new CD3-epsilon binding domain that was tested in the clinic was AMG 330.

Antibody constructs as described in WO 2008/119567 are likely to suffer from rapid clearance from the body; thus, whilst they are able to reach most parts of the body rapidly, and are quick to produce and easy to handle, their in vivo applications may be limited by their brief persistence in vivo. Prolonged administration by continuous intravenous infusion was used to achieve therapeutic effects because of the short in vivo half-life of this relatively small molecule. However, such continuous intravenous infusions are classified as inconvenient for the patients and, thus, in case of more convenient alternative treatment approaches, might hamper the election of the compound demonstrated to be more efficient in the treatment of the respective disease. This led to the development of bispecific therapeutics that retain similar therapeutic efficacy and at the same time have favorable pharmacokinetic properties, including a longer half-life. Therefore, an important further development of the so-called canonical BiTE^{®} molecules was the addition of a further domain that extends or enhances the half-life of the antibody construct. The resulting molecules are also called "HLE" (half-life extended) BiTE^{®} molecules, see e.g. WO 2017/134140.

B cell maturation antigen (BCMA, TNFRSF17, CD269) is a transmembrane protein belonging to the TNF receptor super family. BCMA expression is selectively induced during late stage plasma cell differentiation and is absent on naive and memory B cells. Upon BCMA binding to its ligands, B cell activating factor (BAFF) and a proliferation inducing ligand (APRIL), the survival of the bone marrow plasma cells and plasmablasts is promoted. BCMA does not maintain normal B cell homeostasis, but is required for the survival of long lived plasma cells. Studies in BCMA -/- mice showed that the survival of long lived bone marrow plasma cells was impaired, but B cell development and early humoral immune responses were indistinguishable from wild type mice. The mRNA expression of BCMA is highly elevated in malignant plasma cell disorders. By contrast, mRNA expression in normal tissues is very low and restricted to lymphoid tissues where normal long-lived plasma cells are located. BCMA protein expression is reported to be restricted to plasma cells only. Expression of BCMA is confined to plasma blasts and long-lived plasma cells and cannot be detected on other normal human tissues. BCMA is universally expressed on the cell surface of MM cells and at relatively higher levels on malignant plasma cells than the level observed on normal plasma cells. There is no correlation between BCMA expression and MM disease stage, response to last treatment and time from diagnosis. Neither T cells nor myeloid cells or CD34+ hematopoietic stem cells express BCMA. The selective expression of BCMA makes it a very attractive target for antibody-based and chimeric antigen receptor (CAR)-based therapies.

AMG 420 (formerly BI 836909) is a canonical bispecific T cell engager which binds to BCMA on target cells as well as to CD3-epsilon on T cells. It functions as a bridge between BCMA positive target cells such as MM cells and cytotoxic T lymphocytes (CTLs) by directing the cytolytic activity of CTLs to the target cells. AMG 420 consists of two single chain variable fragments (scFv), one being directed to BCMA and the other one to CD3. Each of the scFv fragments consists of a VH and a VL domain connected with a glycine/serine linker. The two scFv fragments are also connected with a glycine/serine linker. Half-life extended anti-BCMA x anti-CD3 antibody constructs such as AMG 701 are described in detail in WO 2017/134134. CHO S-F et al., CLINICAL LYMPHOMA, MYELOMA AND LEUKEMIA - 17TH INTERNATIONAL MYELOMA WORKSHOP 20191001 ELSEVIER INC. NLD, vol. 19, no. 10, Supplement, 1 October 2019 discloses that AMG 701, a half-life extended BiTE induces T cell-redirected lysis of human multiple myeloma cells. NCT03287908 discloses the outline of a clinical trial with AMG 701 in subjects with multiple myeloma. There is an ongoing unmet medical need for the development of therapies for the treatment of BCMA positive neoplasms, in particular with such half-life extended anti-BCMA x anti-CD3 molecules.

### Summary of the invention

The present invention provides an antibody construct comprising the amino acid sequence as depicted in SEQ ID NO: 661, for use in the treatment or amelioration of a BCMA positive neoplasm in a human subject, wherein the BCMA positive neoplasm is relapsed and/or refractory multiple myeloma, wherein the antibody construct is administered at a minimum dose of 800 µg/day in at least one cycle, wherein one cycle comprises at least three individual administrations of the antibody construct, wherein the antibody construct is administered in one or two dose steps during the first cycle, wherein a first dose is between 800 µg/day and 1200 µg/day, and a last dose (target dose) is between 6500 µg/day and 12 mg/day, and wherein the antibody construct is administered intravenously.

In one embodiment, antibody construct is administered for 2, 3, 4, 5, 6, 7, 8, 9, 10, 11, 12, 13, 14, 15, 16, 17, 18, 19, 20, 21, 22, 23, 24, 25 or more cycles, preferably wherein the antibody construct is administered during the second cycle and optionally during any subsequent cycle at constant doses.

In a further embodiment, one cycle has about 25 to about 30 days, preferably about 26 or 27 to about 29 days, and more preferably about 28 days.

In another embodiment, the first cycle comprises or consists of three to six individual administrations, preferably four or five individual administrations of the antibody construct.

In yet another embodiment, the antibody construct is administered via intravenous bolus injection, bolus infusion or short-term intravenous infusion.

In a further embodiment, the antibody construct has an elimination half-life (T_{1/2}) of about 3 to about 14 days, about 4 to about 12 days, about 3 or 4 to about 10 days, about 3 or 4 to about 8 days, or about 5 to about 7 days.

### Brief description of the drawings

Figure 1 shows an exemplary dose exploration of AMG 701
Figure 2 shows a week 1 step dosing optimization protocol

### Detailed description of the invention

It is hence an object of the present invention to provide an administration scheme for a half-life extended anti-BCMA x anti-CD3 antibody construct which provides for a favorable safety and tolerability profile, while resulting in a positive efficacy signal in BCMA positive neoplasms.

The present invention therefore relates to an antibody construct comprising the amino acid sequence as depicted in SEQ ID NO: 661, for use in the treatment or amelioration of a BCMA positive neoplasm in a human subject, wherein the BCMA positive neoplasm is relapsed and/or refractory multiple myeloma, wherein the antibody construct is administered at a minimum dose of 800 µg/day in at least one cycle, wherein one cycle comprises at least three individual administrations of the antibody construct, wherein the antibody construct is administered in one or two dose steps during the first cycle, wherein a first dose is between 800 µg/day and 1200 µg/day, and a last dose (target dose) is between 6500 µg/day and 12 mg/day, and wherein the antibody construct is administered intravenously.

A "neoplasm" is an abnormal growth of tissue, usually but not always forming a mass. When also forming a mass, it is commonly referred to as a "tumor". In brain tumors, the uncontrolled division of cells means that the mass of a neoplasm increases in size, and in a confined space such as the intracranial cavity this quickly becomes problematic because the mass invades the space of the brain pushing it aside, leading to compression of the brain tissue and increased intracranial pressure and destruction of parenchyma. According to the invention, the term "neoplasm" or "tumor" also refers to a condition that would benefit from treatment with the antibody construct as described herein. This includes chronic and acute disorders or diseases, including those pathological conditions that predispose a mammal to the condition (neoplasm or tumor) in question.

Neoplasms or tumors can be benign, potentially malignant (pre-cancerous), or malignant (cancerous). Malignant neoplasms / tumors are commonly called cancer. They usually invade and destroy the surrounding tissue and may form metastases, i.e., they spread to other parts, tissues or organs of the body. A "primary tumor" is a tumor growing at the anatomical site where tumor progression began and proceeded to yield a cancerous mass. For example, a brain tumor occurs when abnormal cells form within the brain. Most cancers develop at their primary site but then go on to form metastases or spread to other parts (e.g. tissues and organs) of the body. These further tumors are secondary tumors. Most cancers continue to be called after their primary site, even after they have spread to other parts of the body.

Lymphomas and leukemias are hematopoietic or lymphoid neoplasms. Multiple Myeloma (MM), also known as plasma cell myeloma, is a cancer of plasma cells, a type of white blood cells that normally produce antibodies. For the purposes of the present invention, lymphomas and leukemias as well as MM are also encompassed by the terms "tumor", "cancer" or "neoplasm". Lymphoma is a group of blood cancers that develop from lymphocytes (a type of white blood cell). Leukemia is a group of cancers that usually begin in the bone marrow and result in high numbers of abnormal white blood cells. These white blood cells are not fully developed and are called blasts or leukemia cells. Lymphomas and leukemias are a part of the broader group of tumors of the hematopoietic and lymphoid tissues.

For the purposes of the present invention, the terms "neoplasm", "tumor" and "cancer" may be used interchangeably, and they comprise both primary tumors / cancers and secondary tumors / cancers (or "metastases"), as well as mass-forming neoplasms (tumors) and lymphoid neoplasms (such as lymphomas and leukemias), and also MRD.

The term "minimal residual disease" (MRD) refers to the evidence for the presence of small numbers of residual cancer cells that remain in the patient after cancer treatment, e.g. when the patient is in remission (the patient has no symptoms or signs of disease). A very small number of remaining cancer cells usually cannot be detected by routine means because the standard tests used to assess or detect cancer are not sensitive enough to detect MRD. Nowadays, very sensitive molecular biology tests for MRD are available, such as flow cytometry, PCR and next-generation sequencing. These tests can measure minimal levels of cancer cells in tissue samples, sometimes as low as one cancer cell in a million normal cells. In the context of the present invention, the terms "prevention", "treatment" or "amelioration" of a neoplasm are envisaged to also encompass "prevention, treatment or amelioration of MRD", whether the MRD was detected or not.

Is it envisaged that the BCMA positive neoplasm is a B cell neoplasm or a plasma cell neoplasm. B cells, also known as B lymphocytes, are a type of white blood cell of the lymphocyte subtype. They function in the humoral immunity component of the adaptive immune system by secreting antibodies. Additionally, B cells present antigen (they are also classified as professional antigen-presenting cells) and secrete cytokines. In mammals, B cells mature in the bone marrow, which is at the core of most bones. B cells, unlike the other two classes of lymphocytes - T cells and natural killer (NK) cells - express B cell receptors (BCRs) on their cell membrane. BCRs allow the B cell to bind to a specific antigen, against which it will initiate an antibody response. Plasma cells, also called plasma B cells, plasmocytes, or effector B cells, are white blood cells that secrete large volumes of antibodies. They are usually transported by the blood plasma and the lymphatic system. Plasma cells originate in the bone marrow. B cells differentiate into plasma cells that produce antibody molecules closely modelled after the receptors of the precursor B cell. Once released into the blood and lymph, these antibody molecules bind to the target antigen and initiate its neutralization or destruction.

The level of expression of BCMA on the cell surface can be determined e.g. by flow cytometry analysis. The subpopulation of cells (e.g. B cells, plasma cells, MM cells, CD138+ cells) that is selected for analysis of BCMA expression can e.g. be stained with an anti-BCMA antibody, followed by a secondary antibody, and then analyzed in a FACS assay. A BCMA negative cell line (such as K562, A549, TC71, CCRF-CEM) can be used as control. A shift in the FACS assay (with the BCMA negative cell line defining 0% BCMA expression) indicates that the cells to be analyzed are BCMA positive. Different levels of BCMA expression can exist on the surface cells, such as low, medium or high expression. See also Quinn et al., Blood (2011) 117:890-901 and Sanchez et al, Br J Heamatol 2012 Jul 18.

The "BCMA positive neoplasm" or the "(BCMA positive) B cell neoplasm or plasma cell neoplasm" can be selected from the group including, but not limited to, multiple myeloma, relapsed and/or refractory multiple myeloma, heavy chain multiple myeloma, light chain multiple myeloma, extramedullary myeloma (extramedullary plasmacytoma, extramedullary multiple myeloma), plasmacytoma, plasma cell leukemia, Waldenström's macroglobulinemia (lymphoplasmacytic lymphoma), and smoldering myeloma (smoldering multiple myeloma). The present invention relates to an antibody construct for use in the treatment or amelioration of

The antibody construct of the invention is for use in the treatment, amelioration and/or prevention of relapsed and/or refractory multiple myeloma as described herein in a human subject in need thereof. The term "treatment" refers to both therapeutic treatment and prophylactic or preventative measures. Treatment includes the administration of the antibody construct (or the pharmaceutical composition comprising such antibody construct) to the patient's body, to an isolated tissue, or to a cell from a patient or a subject in need who has a BCMA positive neoplasm as described herein, a symptom of such neoplasm, or a predisposition toward such neoplasm, with the purpose to cure, heal, alleviate, relieve, alter, remedy, ameliorate, improve, or affect the BCMA positive neoplasm, one or more symptoms of the BCMA positive neoplasm, or the predisposition toward the disease.

The terms "subject in need", "patient" or those "in need of treatment" include those already with the BCMA positive neoplasm, as well as those in an MRD setting and those in which the neoplasm is to be prevented. The terms include human subjects that receive either prophylactic or therapeutic treatment.

The term "amelioration" as used herein refers to any improvement of the disease state (the disease being a BCMA positive neoplasm) of a patient, by the administration of an antibody construct according to the invention to such patient or subject in need thereof. Such an improvement may be seen as a slowing down the progression or stopping the progression of the disease of the patient, and/or as a decrease in severity of disease symptoms, an increase in frequency or duration of disease symptom-free periods or a prevention of impairment or disability due to the disease.

The term "prevention" as used herein means the avoidance of the occurrence or of the re-occurrence of a disease as specified herein, by the administration of an antibody construct according to the invention to a subject in need thereof.

In the case of Multiple Myeloma, the symptoms and signs vary greatly because many organs can be affected by the disease. The common symptoms of multiple myeloma include elevated calcium levels, renal failure, anemia, and bone lesions (together, "CRAB" features). In advanced MM, bone pain, bleeding, and frequent infections may occur. Complications may also include amyloidosis. The International Myeloma Working Group (IMWG) has established criteria for the diagnosis of MM which teach, in addition to the classic CRAB features, three "myeloma defining events" (MDEs):
- 60% or greater clonal plasma cells on bone marrow examination
- Serum involved / uninvolved free light chain ratio of 100 or greater, provided the absolute level of the involved light chain is at least 100 mg/L (a patient's "involved" free light chain-either kappa or lambda-is the one that is above the normal reference range; the "uninvolved" free light chain is the one that is typically in, or below, the normal range)
- More than one focal lesion on MRI (magnetic resonance imaging) that is at least 5 mm or greater in size

The presence of at least one of these markers is considered sufficient for a diagnosis of multiple myeloma, regardless of the presence or absence of symptoms or CRAB features. See also Palumbo A. J Clin Oncol. 2014 Feb 20; 32(6): 587-600.

Bone pain affects almost 70% of patients and is the most common symptom. It usually involves the spine and ribs. Involvement of the vertebrae may lead to spinal cord compression or kyphosis. Myeloma bone disease is due to the overexpression of receptor activator for nuclear factor κB ligand (RANKL) by bone marrow stroma. RANKL activates osteoclasts, which resorb bone. The resultant bone lesions are lytic (i.e. they cause breakdown) in nature, and are best seen in plain radiographs. The breakdown of bone also leads to the release of calcium into the blood, leading to hypercalcemia and its associated symptoms.

The anemia found in myeloma is usually normocytic and normochromic. It results from the replacement of normal bone marrow by infiltrating tumor cells and inhibition of normal red blood cell production (hematopoiesis) by cytokines.

A bone marrow biopsy can be performed to estimate the percentage of bone marrow occupied by plasma cells. This percentage is used in the diagnostic criteria for MM. Usually, MM patients have ≥ 10% clonal bone marrow plasma cells. Immunohistochemistry (staining particular cell types using antibodies against surface proteins) can detect plasma cells which express immunoglobulin in the cytoplasm and occasionally on the cell surface; for example, myeloma cells are typically positive for the markers CD56, CD38, CD138, CD319, but other markers may be included as well to define or identify MM.

The so-called "paraprotein" (also called myeloma protein, monoclonal protein or M protein) is an abnormal immunoglobulin fragment that is produced in excess by an abnormal monoclonal proliferation of plasma cells, typically in MM. In theory, MM patients can produce all classes of immunoglobulin, but IgG paraproteins are most common, followed by IgA and IgM, while IgD and IgE myeloma are very rare. In addition, antibody light chains and/or heavy chains may be secreted in isolation: kappa or lambda light chains or any of the five types of heavy chains (alpha, gamma, delta, epsilon or my (µ)-heavy chains). This proliferation of the paraprotein has several deleterious effects on the body, including impaired immune function, abnormally high blood viscosity, and kidney damage. Patients without evidence of paraprotein may have "nonsecretory" myeloma (not producing immunoglobulins); they represent approximately 3% of all MM patients. The presence of serum and/or urinary paraprotein is an indicator for MM, except in patients with true nonsecretory MM. Quantitative measurements of the paraprotein in urine and/or serum of a patient can be used to establish a diagnosis and/or to monitor the disease.

Kidney failure may develop both acutely and chronically. The most common cause of kidney failure in MM is due to proteins secreted by the malignant cells. Myeloma cells produce monoclonal proteins of varying types, most commonly immunoglobulins (antibodies) and free light chains, resulting in abnormally high levels of these proteins (paraproteins) in the blood. Depending on the size of these proteins, they may be excreted through the kidneys, but kidneys can also be damaged by their effects. Furthermore, increased bone resorption leads to hypercalcemia and causes nephrocalcinosis, thereby contributing to the kidney failure.

The most common infections occurring in MM are pneumonias and pyelonephritis. The increased risk of infection is due to immune deficiency. Although the total immunoglobulin level is typically elevated in MM, the majority of the antibodies are ineffective monoclonal antibodies from the clonal plasma cell.

It is envisaged that the administration of the antibody construct in the treatment or amelioration of a BCMA positive neoplasm according to the invention
- reduces the level of paraprotein or free light chain in the urine and/or serum by at least about 20%, at least about 30%, at least about 40%, at least about 50%, at least about 60%, at least about 70%, at least about 80%, or at least about 90% relative to the paraprotein or free light chain level in the urine and/or serum, respectively, prior to the start of the treatment, i.e. prior to the first administration of the antibody construct ("prior to", in this specific context, means within 1, 2, 4, 6, 8, or 12 hours before, within 1, 2, 3, 4, 5 or 6 days before, within 1, 2, 3 or 4 weeks before, or within 1, 2, 3 or 4 months before);
- reduces the percentage of plasma cells in the bone marrow by at least about 20%, at least about 30%, at least about 40%, at least about 50%, at least about 60%, at least about 70%, at least about 80%, or at least about 90% relative to the percentage of plasma cells in the bone marrow prior to the start of the treatment, i.e. prior to the first administration of the antibody construct ("prior to", in this specific context, means within 1, 2, 4, 6, 8, or 12 hours before, within 1, 2, 3, 4, 5 or 6 days before, within 1, 2, 3 or 4 weeks before, or within 1, 2, 3 or 4 months before);
- induces a reduction of any of the symptoms described above by at least about 20%, at least about 30%, at least about 40%, at least about 50%, at least about 60%, at least about 70%, at least about 80%, or at least about 90% relative to the symptoms prior to the start of the treatment, i.e. to the first administration of the antibody construct ("prior to", in this specific context, means within 1, 2, 4, 6, 8, or 12 hours before, within 1, 2, 3, 4, 5 or 6 days before, within 1, 2, 3 or 4 weeks before, or within 1, 2, 3 or 4 months before, depending on the symptom);
- inhibits tumor growth or tumor cell proliferation by at least about 20%, at least about 30%, at least about 40%, at least about 50%, at least about 60%, at least about 70%, at least about 80%, or at least about 90% relative to untreated patients or relative to untreated cells; and/or
- induces lysis of the cells of the BCMA positive neoplasm of at least about 20%, at least about 30%, at least about 40%, at least about 50%, at least about 60%, at least about 70%, at least about 80%, or at least about 90% relative to untreated patients or relative to untreated cells.

The ability of an antibody construct of the invention to inhibit tumor growth / tumor cell proliferation or to induce cell lysis may be evaluated in an animal model predictive of efficacy in human tumors, or in an *in vitro* or *ex vivo* study (such as depletion of BCMA positive cells by autologous T cells from a multiple myeloma patient's BM aspirate induced by the antibody construct). Efficacy assessments of the antibody construct may furthermore be performed as follows: Tumor assessment can be done by analysis of percent myeloma involvement, by FISH (fluorescent in situ hybridization) as well as by karyotyping in the bone marrow (BM). Data for BM karyotyping and FISH may be obtained from a BM sample. Serum protein electrophoresis (SPEP) and urine protein electrophoresis (UPEP) allow for measurement of serum / urine M protein. Immunofixation is another means to detect serum and/or urine M protein. It is also envisaged that serum free light chain assay and ratio analysis can be performed. In case of free light chain (FLC) multiple myeloma, FLC can be analyzed in serum and urine (sFLC and uFLC). Levels of involved/uninvolved FLC, ratio of monoclonal lambda-FLC/kappa-FLC, and ratio of monoclonal kappa-FLC/lambda-FLC can be determined. Furthermore, quantitative and qualitative immunoglobulin (Ig) can also be analyzed, and beta-2 microglobulin in serum can be assessed. It is also envisaged that skeletal survey and plasmacytoma assessments can be performed. Screening imaging to evaluate for extramedullary relapse using whole-body MRI or PET/CT can be performed. Imaging appropriate for assessment of bone lesions includes, but is not limited to, CT scan, MRI, PET, PET-CT, or other standard-of-care method. It is also envisaged that minimal residual disease is measured by a next generation sequencing (NGS) based assay. For this purpose, bone marrow aspirates can be collected from subjects suspected to be complete responders. Plasma samples can additionally be collected from subjects at the same time points as BM MRD samples are collected, to assess the feasibility of MRD detection on ctDNA (circulating tumor DNA). MRD response may be defined as <1 tumor cell / 10⁴ normal cells in the bone marrow per FACS using antibodies to cytIgλ, cytIgκ, CD19, CD56 or CD138, CD38, and CD45, as needed. In one embodiment of the invention, these markers are a sufficient condition to define a tumor cell in the context of the present invention.

It is envisaged that a patient's or subject's response to the administration of the antibody construct according to the invention is measured in one of the following ways:
- quantitative measurement of the paraprotein (M protein) or free light chain in the urine and/or serum;
- determination of the percentage of plasma cells in the bone marrow; and/or
- imaging (CT, MRI) of extramedullary manifestations.

The invention provides an antibody construct comprising the amino acid sequence as depicted in SEQ ID NO: 661, for use in the treatment or amelioration of a BCMA positive neoplasm in a human subject, wherein the BCMA positive neoplasm is relapsed and/or refractory multiple myeloma, wherein the antibody construct is administered at a minimum dose of 800 µg/day in at least one cycle, wherein one cycle comprises at least three individual administrations of the antibody construct, wherein the antibody construct is administered in one or two dose steps during the first cycle, wherein a first dose is between 800 µg/day and 1200 ng/day, and a last dose (target dose) is between 6500 ng/day and 12 mg/day, and wherein the antibody construct is administered intravenously.

The antibody construct of the invention is administered in at least one cycle, but more cycles of administration such as 2, 3, 4, 5, 6, 7, 8, 9, 10, 11, 12, 13, 14, 15, 16, 17, 18, 19, 20, 21, 22, 23, 24, 25 or more are also envisaged. The term "at least one cycle" means "one or more cycles". According to the invention, "one cycle" corresponds to a period of about 25 to about 30 or 31 days, of about 26 or 27 to about 29 days, preferably of about 28 days or four weeks. A cycle begins with the first administration of the antibody construct of the invention on "day 1". One cycle may immediately be followed by a subsequent cycle, but an administration-free interval between two cycles is also envisaged. The different administration cycles do not have to be fully identical. In particular, it is envisaged that the first cycle may differ from the following cycles, in particular in terms of the dose of the antibody construct.

The term "a minimum dose of..." means "a dose of at least ...". The dose of the antibody construct is between 800 µg per day and 12 mg per day. It may be 1000 µg, 1200 µg, 1500 µg, 1600 µg, 2000 µg, 2500 µg, 3000 µg, 3500 µg, 4000 µg, 4500 µg, 5000 µg, 5500 µg, 6000 µg, 6500 µg, 7000 µg, 7500 µg, 8000 µg, 8500 µg, 9000 ng, 9500 µg, 10 mg, 11 mg or 12 mg per day.

The dose (the daily dose) of the antibody construct to be administered in the first cycle increases in one or two steps. In this context, "stable" or "stable throughout the cycle" means that the dose to be administered daily (such as 800 µg) is identical / constant in each administration within the cycle. In contrast, a "step" or "dose step" means that there is an increase in the dose (daily dose) to be administered from one administration to the subsequent administration. One dose step within a cycle means that a first dose to be administered on one day is followed by a second (higher) dose to be administered on a subsequent day. Accordingly, two dose steps within a cycle mean that a first dose is followed by a second dose which is higher than the first dose, and the second dose is followed by a third dose which is higher than the second dose. At least the last dose to be administered in the first cycle is the so-called "target dose". The target dose is between 6500 µg per day and 12 mg per day. The target dose may be e.g. 6500 µg, 7000 µg, 7500 µg, 8000 µg, 8500 µg, 9000 µg, 9500 µg, 10 mg, 11 mg or 12 mg per day. It is envisaged that the target dose is reached on day 3, on day 4, on day 5, on day 6, on day 7, on day 8, on day 9 or on day 10 of the first cycle. It is envisaged the first dose to be administered on day 1 is between 800 µg and 1200 µg per day. In case there are two steps in the first cycle, the second dose to be administered is envisaged to be between 2500 µg and 5000 µg per day, preferably about 3000 µg or about 4500 µg per day. In case there are two steps in the first cycle, the second dose is envisaged to be administered on day 3, day 4 or day 5.

One cycle comprises at least three individual administrations of the antibody construct, preferably four individual administrations. This means that the subject in need receives the antibody construct of the invention at least three times during a cycle, preferably four times. For example, if a cycle consists of four weeks, the subject in need may be administered with the antibody construct once per week. An "individual administration" hence means the administration of a specified dose as defined above on one day. Within one cycle, such individual administration is followed by a period (of usually at least one day) in which the antibody construct is not administered. It is also envisaged that the first cycle comprises or consists of three to six individual administrations, preferably four or five individual administrations of the antibody construct. It is envisaged that the last two or three administrations within the first cycle are at the target dose.

In the first cycle, it is envisaged that the antibody construct is administered on day 1 (d1). Further or subsequent days of administration in the first cycle are envisaged to be d3 or d4 or d5. Further days of administration are envisaged to be d7 or d8 or d9. Further days of administration are envisaged to be d11 or d12 or d13. Further days of administration are envisaged to be d14 or d15 or d16. Further days of administration are envisaged to be d18 or d19 or d20. Further days of administration are envisaged to be d21 or d22 or d23.

According to one embodiment, the first cycle begins with the administration of 800 µg of the antibody construct on day 1. According to a further embodiment, the administration of the antibody construct in the first cycle continues as follows:
a) administration of the target dose on day 8 (+/- one or two days), day 15 (+/- one or two days) and day 22 (+/- one or two days), wherein the target dose may be e.g. 6500 µg, 7000 µg, 7500 µg, 8000 µg, 8500 µg, 9000 µg, 9500 µg, 10 mg, 11 mg or 12 mg per day;
b) administration of the target dose on day 4 or day 5 (+/- one or two days), day 12 (+/- one or two days) and day 19 (+/- one or two days), wherein the target dose may be as defined in a);
c) administration of the target dose on day 3 or day 4 (+/- one or two days), day 8 (+/- one or two days), day 15 (+/- one or two days) and day 22 (+/- one or two days), wherein the target dose may be as defined in a); or
d) administration of a second dose (higher than 800 µg per day) on day 3 or day 4 (+/- one or two days), and of the target dose on day 8 (+/- one or two days), day 15 (+/- one or two days) and day 22 (+/- one or two days). The second dose is envisaged to be between 2500 µg and 5000 µg per day, preferably about 3000 µg or about 4500 µg per day, and the target dose may be as defined in a) and must be higher than the second dose.

It is envisaged that the antibody construct is administered during the second cycle and optionally during any subsequent cycle at constant doses (constant daily doses). No dose steps are envisaged for the second cycle and optionally for any subsequent cycle. Beginning with the second cycle, it is envisaged that the antibody construct is administered at the target dose. The target dose is envisaged to be between 6500 µg and 12 mg per day. The target dose may be e.g. 6500 µg, 7000 µg, 7500 µg, 8000 ng, 8500 µg, 9000 ng, 9500 µg, 10 mg, 11 mg or 12 mg per day. It is envisaged that beginning with the second cycle, the antibody construct is administered in weekly intervals. For example, the administration in the second and any subsequent cycle is on d1, d8 (+/- one or two days), d15 (+/- one or two days) and d22 (+/- one or two days).

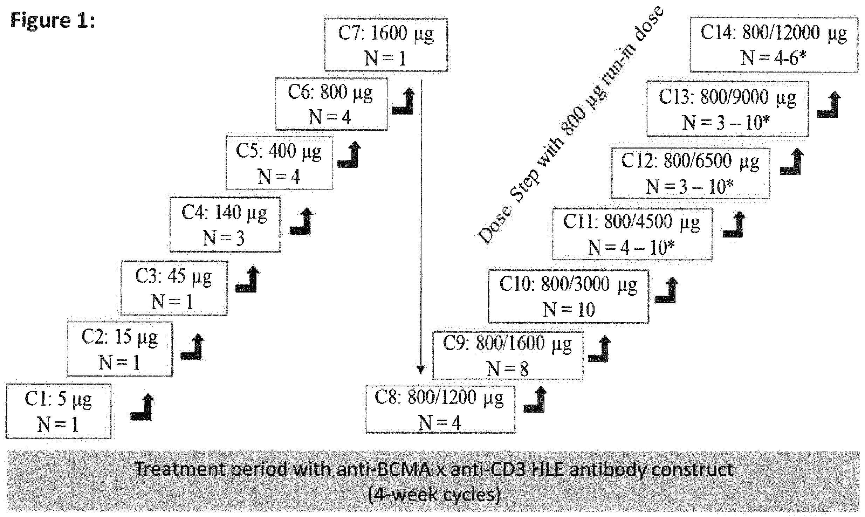

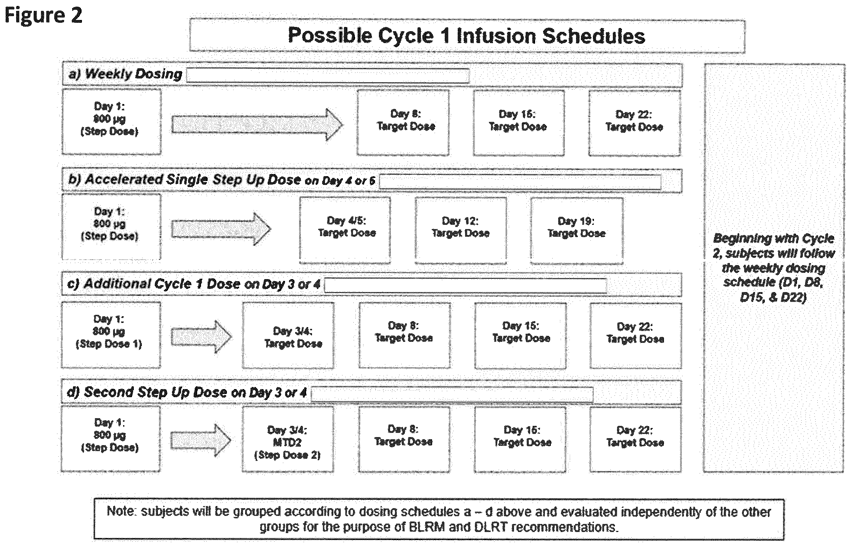

Preferred administration schemes are depicted in Figures 1 and 2.

It is envisaged that the antibody construct of the invention has a molecular weight of about 75 to about 200 kDa, about 80 to about 175 kDa, about 85 to about 150 kDa, about 90 to about 130 kDa, about 95 to about 120 kDa, and preferably about 100 to about 115 kDa or about 105 to about 110 kDa.

The antibody construct of the present invention comprises a domain (the "third domain") which extends or enhances the half-life or the elimination half-life of the construct. The term "extends" or "enhances" may be defined with respect to or in comparison to an antibody construct not comprising such a domain (an "HLE domain"). It is envisaged that the half-life or the elimination half-life of the antibody construct of the invention is at least twice longer than the half-life or the elimination half-life of the antibody construct not comprising the third domain, or at least five times longer, or at least ten times longer, or at least 20 times longer.

The third domain may alternatively be defined as a domain which provides the antibody construct of the invention with a "half-life" or "terminal half-life" or "elimination half-life" (T_{1/2}) of about 3 to about 14 days, about 4 to about 12 days, about 3 or 4 to about 10 days, about 3 or 4 to about 8 days, or about 5 to about 7 days. "Half-life" is the time required for a quantity to reduce to half its initial value. The medical sciences refer to the half-life of substances or drugs (here: the antibody construct) in the human body, e.g. in the serum or in the plasma. Therefore, the "half-life" is sometimes also referred to as "serum half-life" or "plasma half-life". It can be determined by measuring the concentration of the administered antibody construct in the serum / plasma of a subject. Typically, the elimination or removal of an administered substance / drug refers to the body's cleansing through biological processes such as metabolism, excretion, also involving the function of kidneys and liver. "Elimination half-life" may be defined as the time required for the concentration of the drug (here: the antibody construct of the invention) to reach half of its original value.

The antibody construct of the invention will generally be designed for specific routes and methods of administration. A route of administration in pharmacology is the path by which a substance is taken into the body. Routes of administration are generally classified by the location at which the substance is applied, these can be topical (local), enteral (system-wide effect of the substance, but delivered through the gastrointestinal tract), or parenteral (systemic action of the substance, but delivered by routes other than the gastrointestinal tract). In the context of the present invention, the routes of administration are intravenous routes. The reason for the choice of routes of drug administration are governed by various factors such as:
- Physical and chemical properties of the drug. The physical properties are solid, liquid and gas. The chemical properties are solubility, stability, pH, irritancy etc.
- Site of desired action: The action may be localized and approachable or generalized and not approachable.
- Rate of extent of absorption of the drug from different routes.
- Condition of the patient.

The antibody construct of this invention (and a pharmaceutical composition comprising this antibody construct) is particularly useful for parenteral administration. Parenteral administration generally acts more rapidly than topical or enteral administration, and often comes along with a very high bioavailability of up to 100% (in particular, in the case of IV administration). In general, parenteral administration includes, but is not limited to, intravenous (IV), intracerebral, intraarterial, intraperitoneal, intraosseous, and intravesical delivery.

The administration according to the present invention is intravenous. Parenteral or intravenous administration can be performed by injection (e.g. using a needle and a syringe) or by infusion (e.g. via a catheter and a pump system). It is hence envisaged that the administration according to the present invention is via intravenous injection or via intravenous infusion. Usually, an IV infusion is administered via a line, a port or a catheter (small, flexible tube), such as a central venous access or a central venous catheter (CVC) which is a catheter placed into a large vein, or a peripheral venous catheter (PVC), which is a catheter placed into a peripheral vein. In general, catheters or lines can be placed in veins in the neck (internal jugular vein), chest (subclavian vein or axillary vein), groin (femoral vein), or through veins in the arms (also known as a PICC line, or peripherally inserted central catheters). Central IV lines have their catheters that are advanced through a vein and empty into a large central vein, usually the superior vena cava, inferior vena cava or even the right atrium of the heart. A peripheral intravenous (PIV) line is used on peripheral veins (the veins in the arms, hands, legs and feet). A port is a central venous line that does not have an external connector; instead, it has a small reservoir that is covered with silicone rubber and is implanted under the skin. Medication is administered intermittently by placing a small needle through the skin, piercing the silicone, into the reservoir. When the needle is withdrawn, the reservoir cover reseals itself. The cover can accept hundreds of needle sticks during its lifetime.

The present invention provides for a bolus administration of the antibody construct of the invention. A bolus is the administration of a discrete amount of a medication, drug, or other compound within a specific negligible time, generally within 1-30 minutes. The bolus administration is given intravenously. A bolus is usually administered via injection (e.g. an intravenous bolus injection), but a bolus infusion (e.g. an intravenous bolus infusion) is also possible. A short-term infusion is an infusion (usually an IV infusion), of a relatively small volume (such as 50 mL to 500 mL, or 100 mL to 250 mL), which is administered over a period of, at most, three hours, usually of 30 to 60 minutes or about 60 minutes. A short-term (or short-term IV) infusion of the antibody construct is envisaged by the present invention.

Intravenous "intermittent infusion" is an infusion of a volume of medication over a set period of time, such as 20-120 minutes or 30-60 minutes, at prescribed intervals, such as every 4, 6, 8, 10, 12, 14, 16, 18, 20, 22 or 24 hours. The purpose is to administer small amounts of medication at regular intervals. An intermittent medication - like any other form of infusion - may be administered by gravity or via an electronic infusion device (EID), also known as an infusion pump.

In the case of an infusion, an infusion pump may be used to infuse the medication (antibody construct) into a patient's circulatory system. The pump is used intravenously, although arterial and epidural infusions with pumps are also possible. The solution for infusion may be prepared in bags for IV infusion and delivered through infusion lines. Infusion pumps can administer fluids in ways that would be unreliable if performed manually. For example, they can administer as little as 0.1 mL per hour injections, injections every minute, injections with repeated boluses, up to a maximum number per hour, or fluids whose volumes vary by the time of day. It is also possible that infusions are administered using only the pressure supplied by gravity. Different types of infusions according to the present invention include, but are not limited to, bolus infusion, short-term infusion, and intermittent infusion. The antibody construct of the present invention may hence be administered e.g. as a bolus administration (bolus injection or bolus infusion), as an injection, or as a short-term infusion, for example over a period of about 30 to about 90 minutes or about 60 minutes.

Pharmaceutical compositions may be administered using a medical device. Examples of medical devices for administering pharmaceutical compositions are described in U.S. Patent Nos. 4,475,196; 4,439,196; 4,447,224; 4,447, 233; 4,486,194; 4,487,603; 4,596,556; 4,790,824; 4,941,880; 5,064,413; 5,312,335; 5,312,335; 5,383,851; and 5,399,163.

**In** the context of the present invention, it is envisaged that a premedication is administered prior to each administration (or "individual administration") of the antibody construct, in particular during the first cycle. It is envisaged that "prior to", in this specific context, means within 24 hours, 18 hours, twelve hours, six hours, five hours, four hours, or three hours, and preferably within 120, 90, 60 or 30 minutes before the administration of the antibody construct. The premedication may e.g. be administered 30-120 or 30-60 minutes prior to the administration of the antibody construct. It is also envisaged that a comedication is administered concurrent with or after the start of administration of the antibody construct in the first cycle, and optionally also concurrent with or after the start of administration of the antibody construct in one or more of the following cycles, as needed by the patient. It is envisaged that "after", in this specific context, means within 24 hours, 18 hours, twelve hours, six hours, five hours, four hours, or three hours, and preferably within 120, 90, 60, 30, 20, 15 or 10 minutes after the start of administration of the antibody construct. The comedication may e.g. be administered 10-120, 10-60, 10-30 or 15-20 minutes after start of administration of the antibody construct. The purpose of the premedication or comedication may be e.g. to prevent or reduce severity of infusion-related reactions. Premedication is preferred over comedication.

The premedication or comedication may include any one or a combination of the following:
- Paracetamol (acetaminophen, APAP) or an equivalent; to be preferably administered orally (p.o.) or intravenously; and to be preferably administered at a dose of 100-4000 mg, preferably 200-3000 mg or 300-2500 mg or 400-2000 mg or 500-1500 mg, preferably 600-1400 mg, 700-1300 mg, 800-1200 mg, 900-1100 mg or about 1000 mg p.o. paracetamol (or an equivalent dose for an equivalent medication and/or another route of administration). The skilled person knows how to identify paracetamol equivalents. They include, but are not limited to, ibuprofen (to be administered e.g. at a dose of 100-3200 mg, preferably 200-3000 mg or 300-2500 mg or 400-2000 mg, preferably 500-1500 mg, 600-1200 mg, 700-1000 mg, 750-900 mg or about 800 mg) and metamizole (to be administered e.g. at a dose of 100-4000 mg, preferably 200-3000 mg or 300-2500 mg or 400-2000 mg, or about 500-1000 mg)
- One or more analgesics selected from meperidine, dipyrone, hydromorphone, fentanyl, and tramadol
- Antihistamine, to be preferably administered orally or intravenously, and to be preferably administered at a dose equivalent to diphenhydramine 50 mg i.v. The skilled person knows how to identify antihistamines. They include, but are not limited to, antihistamines of oral, parenteral or rectal route such as: azatadine (maximum dose e.g. 4 mg/day), brompheniramine (maximum dose e.g. 30 mg/day), cetirizine (maximum dose e.g. 15 mg/day), chlorpheniramine (maximum dose e.g. 30 mg/day), clemastine (maximum dose e.g. 10 mg/day), cyproheptadine (maximum dose e.g. 15 mg/day), desloratadine (maximum dose e.g. 7 mg/day), dexchlorpheniramine (maximum dose e.g. 15 mg/day), diphenhydramine (maximum dose e.g. 350/per day), doxylamine (maximum dose e.g. 180 mg/day), fexofenadine (maximum dose e.g. 200 mg/day), loratadine (maximum dose e.g.15 mg/day), phenindamine (maximum dose e.g. 180 mg/day)
- Glucocorticoid, to be preferably administered orally or intravenously, and to be preferably administered at a dose equivalent to 2-20 mg or 4-16 mg or 6-12 mg or 8 mg dexamethasone i.v. (the equivalence referring to the glucocorticoid potency)

All four substances or substance groups listed above may be administered as premedication or comedication, or a combination of only two or a combination of three substances, or only one of the four substances. It is envisaged that the glucocorticoid (GC) dose administered before the start of the second cycle may be identical to the GC dose administered before start of the first cycle, or may be reduced to about 50% of the dose administered before start of the first cycle, or may be omitted for the second (and/or potentially any subsequent) cycle. A reduction of the GC dosage may apply e.g. if the antibody construct according to the invention is well tolerated without significant signs of infusion-related reactions during the first cycle. It is furthermore envisaged that the dose may further be reduced before start of the third and any subsequent cycle. Alternatively, while GC is administered as premedication (and potentially comedication) before the start of the first cycle, no GC premedication or comedication is administered in the second, third, fourth and/or fifth cycle. In general, the dose of the premedication or comedication that is to be used in accordance with the embodiments of the present invention will depend on the circumstances of the individual patient.

Glucocorticoids are a class of corticosteroids, which are a class of steroid hormones. Glucocorticoids are corticosteroids that bind to the glucocorticoid receptor. A less common synonym is glucocorticosteroid. Cortisol (known as hydrocortisone when used as a medication) is the most important human glucocorticoid. A variety of synthetic glucocorticoids, some far more potent than cortisol, have been created for therapeutic use. They differ in both pharmacokinetics (e.g. absorption factor, half-life, volume of distribution, clearance) and pharmacodynamics (e.g. glucocorticoid potency or mineralocorticoid potency). Cortisol is the standard of comparison for glucocorticoid potency. One example for commonly prescribed replacement steroid equivalents may be prednisone (5 mg) = cortisone (25 mg) = dexamethasone (0.75 mg) = hydrocortisone (20 mg) = methylprednisolone (4 mg). These doses indicate the equivalent pharmacologic dose of systemic glucocorticoids. Another corticosteroid comparison chart indicating the half-lives of the different substances can be found e.g. in www.nadf.us/downloads/adrenalhormone.pdf. Corticosteroid conversion calculators or equivalency tables are available in the internet.

Examples of GCs to be used as premedication or comedication in the present embodiment include, but are not limited to, cortisone, hydrocortisone, prednisone, prednisolone, methylprednisolone, dexamethasone, betamethasone, beclomethasone, budesonide, triamcinolone, cloprednol, deflazacort, fluocortolone, cortivazol, paramethasone, fluticasone, fluticasone propionate, triamcinolone acetonide, as well as combinations and/or pharmaceutically acceptable derivatives thereof. In the context of the present invention, the different GCs may be used alone or in combination. Dexamethasone, prednisone and prednisolone are preferred embodiments of GCs.

It is envisaged that all substances which already are or will be classified as a "glucocorticoid" may be employed in the context of the present invention as well. Such future glucocorticoids include compounds which specifically bind to and activate the glucocorticoid receptor. The term "specifically binds to the GC receptor" means in accordance with the present invention that the GC (or a compound which is assumed to act like a GC) associates with (e.g., interacts with) the GC receptor (also known as NR3C1) to a statistically significant degree as compared to association with proteins/receptors generally (i.e., non-specific binding). When the GC receptor binds to glucocorticoids, its primary mechanism of action is the regulation of gene transcription. In the absence of GC, the glucocorticoid receptor (GR) resides in the cytosol complexed with a variety of proteins including heat shock protein 90 (hsp90), heat shock protein 70 (hsp70) and the protein FKBP52 (FK506-binding protein 52). Binding of the GC to the glucocorticoid receptor results in release of the heat shock proteins. It is thus envisaged that a future GC, or a pharmaceutically acceptable derivative or salt of a GC, is able to bind to the GC receptor and to release the above mentioned heat shock proteins. The activated GR complex then up-regulates the expression of anti-inflammatory proteins in the nucleus or represses the expression of pro-inflammatory proteins in the cytosol by preventing the translocation of other transcription factors from the cytosol into the nucleus.

The antibody construct of the present invention comprises a first domain which binds to BCMA, a second domain which binds to CD3 and a third domain which extends (or enhances) the half-life of the antibody construct as defined in the claims.

The term "antibody construct" refers to a molecule in which the structure and/or function is/are based on the structure and/or function of an antibody, e.g., of a full-length immunoglobulin molecule. An antibody construct hence immunospecifically binds to its target or antigen, and/or it comprises domains which are derived from or which are the heavy chain variable region (VH) and/or the light chain variable region (VL) of an antibody. Furthermore, an antibody construct according to the invention comprises the minimum structural requirements of an antibody which allow for immunospecific target binding. This minimum requirement is defined by the presence of at least three light chain CDRs (i.e. CDR1, CDR2 and CDR3 of the VL region) and three heavy chain CDRs (i.e. CDR1, CDR2 and CDR3 of the VH region)

"Antibody constructs" of the present comprise modified fragments of antibodies, also called antibody variants or antibody derivatives. The modified fragments include scFv domains. The antibody constructs according to the invention are scFc constructs.

Furthermore, the definition of the term "antibody construct" includes bivalent and polyvalent / multivalent constructs, which specifically bind to two, three or more antigenic structures, through distinct binding domains. An antibody construct can hence have more binding valences than specificities, e.g. in a case where it has two binding domains for the first target (BCMA) and one binding domain for the second target (CD3) - or vice versa - in which case the construct is trivalent and bispecific. Moreover, the definition of the term "antibody construct" includes molecules consisting of only one polypeptide chain ("single-chain polypeptide") as well as molecules consisting of two, three, four or more polypeptide chains, which chains can be either identical (homodimers, homotrimers or homo oligomers) or different (heterodimer, heterotrimer or heterooligomer). Examples for the above identified antibodies and their fragments, variants, derivatives and antibody constructs derived therefrom are described *inter alia* in Harlow and Lane, Antibodies: A laboratory manual, CSHL Press (1988); Kontermann and Dübel, Antibody Engineering, Springer, 2nd ed. 2010; and Little, Recombinant Antibodies for Immunotherapy, Cambridge University Press 2009. The antibody construct of the present invention is a single chain antibody construct / a single chain polypeptide.

The term "binding domain" or "domain which binds to..." characterizes in connection with the present invention a domain of the antibody construct which immunospecifically binds to / interacts with / recognizes a given epitope on the target or antigen (here: BCMA in the case of the first domain, and CD3 in the case of the second domain). The structure and function of the first domain (binding to BCMA), and also the structure and function of the second domain (binding to CD3), are based on the structure and function of an antibody, e.g. of a full-length immunoglobulin molecule. The "binding domain" or "domain which binds to..." may-hence comprise the minimum structural requirements of an antibody which allow for immunospecific target binding. This minimum structural requirement of the first domain are defined by the presence of three light chain CDRs (i.e. CDR1, CDR2 and CDR3 of the VL region) and/or of three heavy chain CDRs (i.e. CDR1, CDR2 and CDR3 of the VH region) The second domain also comprises this minimum structural requirement of an antibody which allow for the immunospecific target binding. The second domain also comprises three light chain CDRs (i.e. CDR1, CDR2 and CDR3 of the VL region) and/or three heavy chain CDRs (i.e. CDR1, CDR2 and CDR3 of the VH region) A "domain which binds to" (or a "binding domain") comprises an antibody light chain variable region (VL) and an antibody heavy chain variable region (VH) Fd fragments, for example, often retain some antigen-binding function of the intact antigen-binding domain.

The format of a "domain which binds to" (or a "binding domain") is a single chain Fv (scFv).

The terms "(specifically or immunospecifically) binds to", "(specifically or immunospecifically) recognizes", or "(specifically or immunospecifically) reacts with" mean in accordance with this invention that an antibody construct or a binding domain interacts or (immuno-)specifically interacts with a given epitope on the target molecule (antigen), here: BCMA and CD3, respectively. This interaction or association occurs more frequently, more rapidly, with greater duration, with greater affinity, or with some combination of the aforementioned, to an epitope on the specific target than to alternative substances (non-target molecules). Because of the sequence similarity between homologous proteins in different species, an antibody construct or a binding domain that immunospecifically binds to its target (such as a human target) may, however, cross-react with homologous target molecules from different species (such as, from non-human primates, e.g. macaque). The term "specific / immunospecific binding" can hence include the binding of an antibody construct or binding domain to epitopes or structurally related epitopes in more than one species.

In the context of the present invention, the term "epitope" refers to the part or region of the antigen that is recognized / immunospecifically recognized by the binding domain. An "epitope" is antigenic, and thus the term epitope is sometimes also referred to as "antigenic structure" or "antigenic determinant". The part of the binding domain that binds to the epitope is called a paratope. Specific binding is believed to be accomplished by specific motifs in the amino acid sequence of the binding domain and the antigen. Thus, binding is achieved as a result of their primary, secondary and/or tertiary structure as well as the result of potential secondary modifications of said structures. The specific interaction of the paratope with its antigenic determinant may result in a simple binding of said site to the antigen. In some cases, the specific interaction may alternatively or additionally result in the initiation of a signal, e.g. due to the induction of a change of the conformation of the antigen, an oligomerization of the antigen, etc.

The epitopes of protein antigens are divided into two categories, conformational epitopes and linear epitopes, based on their structure and interaction with the paratope. A conformational epitope is composed of discontinuous sections of the antigen's amino acid sequence. These epitopes interact with the paratope based on the three-dimensional surface features and shape or tertiary structure (folding) of the antigen. Methods of determining the conformation of epitopes include, but are not limited to, x-ray crystallography, two-dimensional nuclear magnetic resonance (2D-NMR) spectroscopy and site-directed spin labelling and electron paramagnetic resonance (EPR) spectroscopy. By contrast, linear epitopes interact with the paratope based on their primary structure. A linear epitope is formed by a continuous sequence of amino acids from the antigen and typically includes at least 3 or at least 4, and more usually, at least 5 or at least 6 or at least 7, for example, about 8 to about 10 amino acids in a unique sequence.

The interaction between the binding domain and the epitope of the target antigen implies that a binding domain exhibits appreciable or significant affinity for the epitope / the target antigen (here: BCMA and CD3, respectively). In general, it does furthermore not exhibit significant affinity for proteins or antigens other than the target antigen (here: BCMA / CD3) - notwithstanding the above discussed cross-reactivity with homologous targets e.g. from other species. "Significant affinity" includes binding with an affinity (dissociation constant, KD) of ≤10⁻⁶ M. Preferably, binding is considered specific when the binding affinity is ≤10⁻⁷ M, ≤10⁻⁸ M, ≤10⁻⁹ M, ≤10⁻¹⁰ M, or even ≤10⁻¹¹ M, or ≤10⁻¹² M. Whether a binding domain (immuno-)specifically reacts with or binds to a target can be tested readily e.g. by comparing the affinity of said binding domain to its desired target protein or antigen with the affinity of said binding domain to non-target proteins or antigens (here: proteins other than BCMA or CD3, respectively). Preferably, an antibody construct of the invention does not significantly bind to proteins or antigens other than BCMA or CD3, respectively (*i.e*., the first domain does not bind to proteins other than BCMA and the second domain does not bind to proteins other than CD3). For example, it is envisaged that the antibody construct of the invention (and more specifically its first domain) does not significantly bind to, interact with, recognize or cross-react with human BAFF-R and/or human TACI.

The equilibrium dissociation constant (KD) of an antibody construct of the invention to BCMA can be determined by Scatchard or by biacore analysis, as described e.g. in WO 2013/072406. The KD values for CD3 can e.g. be determined by surface plasmon resonance analysis, as described e.g. in WO 2013/072406. It is envisaged that the antibody construct of the present invention has a KD value for BCMA and/or for CD3 in the 2-digit or 1-digit nanomolar range or in the three digit or even two digit picomolar range.

The term "does not significantly bind" means that an antibody construct or binding domain of the present invention does not bind a protein or antigen other than BCMA or CD3, *i.e*., shows reactivity of ≤30%, preferably ≤20%, more preferably ≤10%, particularly preferably ≤9%, 8%, 7%, 6% or 5% with proteins or antigens other than BCMA or CD3, whereby binding to BCMA or CD3, respectively, is set to be 100%.

The antibody construct of the present invention comprises a first a second domain are-in the format of an scFv (single-chain variable fragment-). An scFv is not actually a fragment of an antibody, but instead is a fusion protein of the variable regions of the heavy (VH) and light chains (VL) of immunoglobulins, connected with a short linker peptide. The linker is usually rich in glycine for flexibility, as well as serine or threonine for solubility. The scFv is designed to retain the specificity of the original immunoglobulin, despite removal of the constant regions and the introduction of the linker. In an scFv, the VH region and the and VL region are arranged in the order VH-VL or VL-VH (from N- to C-terminus). It is envisaged that the VH and the VL regions of the first and the second binding domain are connected via a peptide linker. In the first and the second domain, the VH-region is positioned N-terminally of the linker, and the VL-region is positioned C-terminally of the linker. The first domain and the second domain of the antibody construct are connected via a peptide linker shown in SEQ ID NOs: 689. In the present context, a "short" linker has between 2 and 50 amino acids, preferably between 3 and 35, between 4 and 30, between 5 and 25, between 6 and 20 or between 6 and 17amino acids. The linker between two variable regions of one binding domain is shown in in SEQ ID NOs: 694.

The antibody construct of the present invention is defined in the claims and can be described in that
a) the antibody construct is a single chain polypeptide,
b) the first domain is in the format of an scFv,
c) the second domain is in the format of an scFv, and
d) the first and the second domain are connected via a glycine/serine linker.

The first domain of the antibody construct of the invention binds to BCMA (B cell maturation antigen, TNFRSF17, CD269). on the surface of a target cell. The "target cell" is a cell of a BCMA positive neoplasm as defined in the claims. The first domain binds to human BCMA on the surface of a target cell. It is also envisaged that the first domain binds to macaque BCMA, preferably to macaque BCMA on the surface of a target cell. A preferred amino acid sequence for human BCMA is depicted in SEQ ID NO: 647, for macaque BCMA in SEQ ID NO: 648, for the extracellular domain of human BCMA in SEQ ID NO: 649, for the extracellular domain of macaque BCMA in SEQ ID NO: 650.

The first domain of the antibody construct binds to epitope cluster 3 of human BCMA. A preferred amino acid sequence for epitope cluster 3 of human BCMA is depicted in SEQ ID NO: 651. Antibody constructs having domains that bind to said epitope cluster 3 of BCMA are described in detail in WO 2013/072406. These antibody constructs are shown in WO 2013/072406 to have a very beneficial epitope / activity relationship.

A method for BCMA epitope mapping is used in WO 2013/072406 and described in the following: One or more pre-defined regions (each in the form of a contiguous amino acid stretch) within the extracellular domain of human BCMA is exchanged / replaced with the corresponding region of a rodent BCMA molecule (such as murine BCMA, but other rodent species are also conceivable, so long as the binding domain is not cross-reactive with the rodent species used). These human BCMA/ rodent (murine) BCMA chimeras are expressed on the surface of host cells (such as CHO cells). Binding of the antibody or antibody construct can be tested via FACS analysis. When the binding of the antibody or antibody construct to the chimeric molecule is entirely abolished, or when a significant binding decrease is observed, it can be concluded that the region of human BCMA which was removed from this chimeric molecule is relevant for the immunospecific epitope-paratope recognition. Said decrease in binding is preferably at least 10%, 20%, 30%, 40%, or 50%; more preferably at least 60%, 70%, or 80%, and most preferably 90%, 95% or even 100% in comparison to the binding to human (wild-type) BCMA, whereby binding to human BCMA is set to be 100%. Alternatively or additionally, the above described epitope mapping analysis can be modified by introducing one or more point mutations into the sequence of the extracellular domain of BCMA.

The antibody construct of the present invention is defined in the claims. The first domain of the antibody construct, which binds to BCMA, comprises a VH region comprising

CDR-H1 as depicted in SEQ ID NO: 171, CDR-H2 as depicted in SEQ ID NO: 172, CDR-H3 as depicted in SEQ ID NO: 173 and a VL region comprising CDR-L1 as depicted in SEQ ID NO: 174, CDR-L2 as depicted in SEQ ID NO: 175, and CDR-L3 as depicted in SEQ ID NO: 176.

The first domain comprises a VH region having an amino acid sequence as depicted in SEQ ID NO: 177 and a VL region having an amino acid sequence as depicted in SEQ ID NO: 178. The antibody construct of the present invention comprises first domain which binds to BCMA comprising a polypeptide having an amino acid sequence as depicted in SEQ ID NO: 179.

"T cells" or T lymphocytes are a type of lymphocyte (itself a type of white blood cell) that play a central role in cell-mediated immunity. There are several subsets of T cells, each with a distinct function. T cells can be distinguished from other lymphocytes, such as B cells and NK cells, by the presence of a T cell receptor (TCR) on the cell surface. The TCR is responsible for recognizing antigens bound to major histocompatibility complex (MHC) molecules and is composed of two different protein chains. In 95% of the T cells, the TCR consists of an alpha (α) and beta (β) chain. When the TCR engages with antigenic peptide and MHC (peptide / MHC complex), the T lymphocyte is activated through a series of biochemical events mediated by associated enzymes, co-receptors, specialized adaptor molecules, and activated or released transcription factors.

"CD3" (cluster of differentiation 3) is a T cell co-receptor composed of four chains. In mammals, the CD3 protein complex contains a CD3γ (gamma) chain, a CD3δ (delta) chain, and two CD3ε (epsilon) chains. These four chains associate with the T cell receptor (TCR) and the so-called ζ (zeta) chain to for the "T cell receptor complex" and to generate an activation signal in T lymphocytes. The CD3γ (gamma), CD3δ (delta), and CD3ε (epsilon) chains are highly related cell-surface proteins of the immunoglobulin superfamily and each contain a single extracellular immunoglobulin domain. The intracellular tails of the CD3 molecules contain a single conserved motif known as an immunoreceptor tyrosine-based activation motif (ITAM), which is essential for the signaling capacity of the TCR. The CD3 epsilon molecule is a polypeptide which in humans is encoded by the *CD3E* gene which resides on chromosome 11.

The redirected lysis of target cells via the recruitment of T cells by an antibody construct which binds to CD3 on the T cell and to a target protein on the target cell generally involves cytolytic synapse formation and delivery of perforin and granzymes. The engaged T cells are capable of serial target cell lysis, and are not affected by immune escape mechanisms interfering with peptide antigen processing and presentation, or clonal T cell differentiation; see, for example, WO 2007/042261.

Cytotoxicity mediated by BCMAxCD3 antibody constructs can be measured in various ways. The "half maximal effective concentration" (EC₅₀) is commonly used as a measure of potency of a biologically active molecule such as an antibody construct of the present invention. It is expressed in molar units. In the present case of measuring cytotoxicity, the EC₅₀ value refers to the concentration of an antibody construct inducing a cytotoxic response (lysis of target cells) halfway between the baseline and the maximum. Effector cells in a cytotoxicity assay can *e.g.* be stimulated enriched (human) CD8 positive T cells or unstimulated (human) peripheral blood mononuclear cells (PBMC). The target cells should express BCMA on the cell surface. Preferably the target cells express (at least) the extracellular domain of BCMA on the cell surface. Target cells can be a cell line (such as CHO) which is stably or transiently transfected with BCMA, e.g. human BCMA. Alternatively, the target cells can be a BCMA positive natural expresser cell line, such as the human multiple myeloma cell line L363 or NCI-H929.

The effector to target cell (E:T) ratio in a cytotoxicity assay is usually about 10:1, but can also vary. Cytotoxic activity of BCMAxCD3 antibody constructs can be measured in a 51-chromium release assay (*e.g.* with an incubation time of about 18 hours) or in a in a FACS-based cytotoxicity assay (*e.g.* with an incubation time of about 48 hours). Modifications of the incubation time (cytotoxic reaction) are also envisaged. Other methods of measuring cytotoxicity are well-known and comprise MTT or MTS assays, ATP-based assays including bioluminescent assays, the sulforhodamine B (SRB) assay, WST assay, clonogenic assay and the ECIS technology.

According to one embodiment, the cytotoxic activity mediated by BCMAxCD3 antibody constructs of the present invention is measured in a FACS-based cytotoxicity assay. It is envisaged that the EC₅₀ value of the BCMAxCD3 antibody constructs is ≤5000 pM or ≤4000 pM, more preferably ≤3000 pM or ≤2000 pM, even more preferably ≤1000 pM or ≤500 pM, even more preferably ≤400 pM or ≤300 pM, even more preferably ≤200 pM, even more preferably ≤100 pM, even more preferably ≤50 pM, even more preferably ≤20 pM or ≤10 pM, and most preferably ≤5 pM. It is also envisaged that the antibody construct of the present invention has an EC50 value in the 3-digit, 2-digit or 1-digit pg/ml range, as determined in a FACS-based cytotoxicity assay. The assay may be carried out with the L363 or NCI-H929 cell line or with BCMA transfected CHO cells as target cells and stimulated enriched (human) CD8 positive T cells or unstimulated (human) PBMC as effector cells. See also WO 2013/072406.

The second domain of the antibody construct of the invention binds to human CD3 epsilonon the surface of a T cell. A preferred amino acid sequence for the extracellular domain of human CD3 epsilon is depicted in SEQ ID NO: 653.

The second domain of the antibody construct binds to human CD3 epsilon (or human CD3 epsilon on the surface of a T cell) and to *Callithrix jacchus* or *Saimiri sciureus* CD3 epsilon. The second domain binds to an extracellular epitope of human CD3 epsilon. It is also envisaged that the second domain binds to an extracellular epitope of the human and the *Macaca* CD3 epsilon chain. One epitope of CD3 epsilon is comprised within amino acid residues 1-27 of the human CD3 epsilon extracellular domain (see SEQ ID NO: 654). Even more specifically, the epitope comprises at least the amino acid sequence Gln-Asp-Gly-Asn-Glu. *Callithrix jacchus* is a new world primate belonging to the family of *Callitrichidae*, while *Saimiri sciureus* is a new world primate belonging to the family of *Cebidae.* Binders having such characteristics are described in detail in WO 2008/119567.

Antibodies or bispecific antibody constructs directed against (human) CD3 or specifically against CD3 epsilon are known in the art, and their CDRs, VH and VL sequences can serve as a basis for the second binding domain of an antibody construct. For example, Kung et al. reported in 1979 the development of OKT3 (Ortho Kung T3), the first mAb recognizing CD3 (specifically, the epsilon chain of CD3) on human T cells. OKT3 (muromonab) was the first monoclonal antibody of murine origin to become available for therapy in humans. Newer anti-CD3 monoclonal antibodies include otelixizumab (TRX4), teplizumab (MGA031), foralumab and visilizumab, all targeting the epsilon chain of CD3. Bispecific antibody constructs directed against a (cancer) target and CD3 are also being developed and (pre-)clinically tested, and their CD3 binding domain (CDRs, VH, VL) may serve as a basis for the second binding domain of an antibody construct. Examples include, but are not limited to, Blinatumomab, Solitomab (MT110, AMG 110), Catumaxomab, Duvortuxizumab, Ertumaxomab, Mosunetuzumab, FBTA05 (Bi20, TPBs05), CEA-TCB (RG7802, RO6958688), AFM11, and MGD006 (S80880). Other examples of CD3 binding domains are disclosed e.g. in US 7,994,289 B2, US 7,728,114 B2, US 7,381,803 B1, US 6,706,265 B1.

The antibody construct of the present invention as defined in the claims comprises a second domain which binds to CD3 comprising a VL region comprising

CDR-L1 as depicted in SEQ ID NO: 633, CDR-L2 as depicted in SEQ ID NO: 634, CDR-L3 as depicted in SEQ ID NO: 635 and a VH region comprising CDR-H1 as depicted in SEQ ID NO: 636, CDR-H2 as depicted in SEQ ID NO: 637, and CDR-H3 as depicted in SEQ ID NO: 638.

The second domain which binds to CD3 comprises a VL region as depicted in SEQ ID NO: 641 and a VH region as depicted in SEQ ID NO: 639

The second domain which binds to CD3 consists of a polypeptide having an amino acid sequence depicted in SEQ ID NO: 642.

The antibody construct of the present invention comprises a polypeptide having an amino acid sequence as depicted in SEQ ID NO: 180.

It is also envisaged that the antibody construct of the present invention comprises a polypeptide which has an amino acid sequence depicted in SEQ ID NO: 180 which is linked at its N-terminus with a protein purification tag, preferably via a peptide bond (amide bond). The linking of the protein purification tag at the C-terminus of the polypeptide is preferred. It is envisaged that the protein purification tag is a short peptide. For example, the length of the short peptide may be 2-30 amino acids, 4-25 amino acids, 5-20 amino acids or 6-19 amino acids. Examples of protein purification tags include, but are not limited to, AU1 epitope (e.g. as depicted in SEQ ID NO: 666), AU5 epitope (e.g. as depicted in SEQ ID NO: 667), T7-tag (e.g. as depicted in SEQ ID NO: 668), V5-tag (e.g. as depicted in SEQ ID NO: 669), B-tag (e.g. as depicted in SEQ ID NO: 670), E2 epitope (e.g. as depicted in SEQ ID NO: 671), FLAG epitope / FLAG tag (e.g. as depicted in SEQ ID NO: 672), Glu-Glu tag (e.g. as depicted in SEQ ID NOs: 673 or 674), HA tag, Histidine affinity tag (e.g. as depicted in SEQ ID NO: 675), HSV epitope (e.g. as depicted in SEQ ID NO: 676), KT3 epitope (e.g. as depicted in SEQ ID NO: 677), Myc epitope (e.g. as depicted in SEQ ID NO: 678), polyarginine tag (5-6 Arg residues), polyaspartate tag (5-16 Asp residues), polyhistidine tag (2-10 His residues, usually 6 His residues, see e.g. SEQ ID NOs: 662-665), polyphenylalanine tag (usually 11 Phe residues), S1 tag (e.g. as depicted in SEQ ID NO: 679), S-tag (e.g. as depicted in SEQ ID NO: 680), Strep-tag (e.g. as depicted in SEQ ID NOs: 681 or 682), universal tag (e.g. as depicted in SEQ ID NO: 683), VSV-G (e.g. as depicted in SEQ ID NO: 684), Protein C (e.g. as depicted in SEQ ID NO: 685), and Protein A. A histidine tag is preferred, especially a 6x His tag (SEQ ID NO: 663).

Whether or not an antibody, antibody construct or binding domain binds to the same epitope of BCMA / BCMA on the surface of a target cell as another given antibody, antibody construct or binding domain can be measured by different analyses, *e.g*. by epitope mapping with chimeric or mutated BCMA molecules, as described in WO 2013/072406. Another possibility to identify the epitope within a target is an Alanine scanning assay (see e.g. Morrison KL & Weiss GA. Curr Opin Chem Biol. 2001 Jun;5(3):302-7), where each residue within the target (here: BCMA) to be analyzed is replaced by alanine, e.g. via site-directed mutagenesis. Alanine is used because of its non-bulky, chemically inert, methyl functional group that nevertheless mimics the secondary structure references that many of the other amino acids possess. Sometimes bulky amino acids such as valine or leucine can be used in cases where conservation of the size of mutated residues is desired. Alanine scanning is usually accomplished by site-directed mutagenesis or randomly by creating a PCR library. Furthermore, computational methods to estimate thermodynamic parameters based on theoretical alanine substitutions have been developed. The data can be tested by IR, NMR Spectroscopy, mathematical methods, bioassays, etc. The same analysis can of course be applied for other targets such as CD3.

Whether or not an antibody or antibody construct competes for binding to BCMA / BCMA on the surface of a target cell with another given antibody or antibody construct can be measured in a competition assay such as a competitive ELISA or a cell-based competition assay (using either cells that naturally express BCMA or cells that were stably or transiently transformed with BCMA). Avidin-coupled microparticles (beads) can also be used. Similar to an avidin-coated ELISA plate, when reacted with a biotinylated protein, each of these beads can be used as a substrate on which an assay can be performed. Antigen is coated onto a bead and then precoated with the first antibody. The second antibody is added, and any additional binding is determined. Read-out occurs via flow cytometry. The term "competes for binding", in the present context, means that competition occurs between the two tested antibodies of at least 20%, at least 30%, at least 40%, at least 50%, at least 60%, at least 70%, at least 80% or at least 90%, as determined by any one of the assays disclosed above. The same analysis can of course be applied for other targets such as CD3.

The antibody construct described herein comprises a third domain which extends or enhances the half-life (or "serum half-life") of the antibody construct. Examples for means or domains to extend serum half-life of the antibody constructs of the invention include peptides, proteins or domains of proteins, which are fused or otherwise attached to the antibody constructs. The group of peptides, proteins or protein domains includes peptides binding to other proteins with preferred pharmacokinetic profile in the human body such as serum albumin (see WO 2009/127691). An alternative concept of such half-life extending peptides includes peptides binding to the neonatal Fc receptor (FcRn, see WO 2007/098420), which can also be used for the antibody constructs of the present invention. The concept of attaching larger domains of proteins or complete proteins includes the fusion of human serum albumin, variants or mutants of human serum albumin (see WO 2011/051489, WO 2012/059486, WO 2012/150319, WO 2013/135896, WO 2014/072481, WO 2013/075066) or domains thereof, as well as the fusion of an immunoglobulin constant region (Fc domain) and variants thereof. Such variants of Fc domains are called Fc-based domains and may be further optimized / modified in order to allow the desired pairing of dimers or multimers, to abolish Fc receptor binding (e.g. to avoid ADCC or CDC) or for other reasons. A further concept known in the art to extend the half-life of substances or molecules in the human body is the pegylation of those molecules (such as the antibody constructs of the present invention).

The antibody constructs according to the invention is linked (via peptide bond) with a fusion partner for the purpose of extending the construct's The fusion partners is linked to the C-terminus of the antibody constructs according to the invention through a peptide linker depicted in SEQ ID NOs: 686.

The third domain of the antibody construct of the invention comprises the amino acid sequences depicted in SEQ ID NO: 700. The first and second domains of the antibody construct of the invention are fused to the third domain via a peptide linker as depicted in SEQ ID NO: 686.

In line with the present invention, a "hinge" is an IgG hinge region. This region can be identified by analogy using the Kabat numbering, see e.g. Kabat positions 223-243. In line with the above, the minimal requirement for a "hinge" are the amino acid residues corresponding to the IgG₁ sequence stretch of D231 to P243 according to the Kabat numbering. The terms "CH2" and "CH3" (or "CH2 domain" and "CH3 domain") refer to the immunoglobulin heavy chain constant regions 2 and 3. These regions can as well be identified by analogy using the Kabat numbering, see e.g. Kabat positions 244-360 for CH2 and Kabat positions 361-478 for CH3. It is understood that there is some variation between the immunoglobulins in terms of their IgG₁ Fc region, IgG₂ Fc region, IgG₃ Fc region, IgG₄ Fc region, IgM Fc region, IgA Fc region, IgD Fc region and IgE Fc region (see, e.g., Padlan, Molecular Immunology, 31(3), 169-217 (1993)). The term Fc region refers to the last two heavy chain constant regions of IgA, IgD, and IgG, and the last three heavy chain constant regions of IgE and IgM. The Fc region can also include the flexible hinge N-terminal to these domains. For IgA and IgM, the Fc region may include the J chain. For IgG, the Fc region comprises immunoglobulin domains CH2 and CH3 and the hinge between the first two domains and CH2. Although the boundaries of the Fc region of an immunoglobulin may vary, an example for a human IgG heavy chain Fc portion comprising a functional hinge, CH2 domain and CH3 domain can be defined e.g. to comprise residues D231 (of the hinge domain) to P476 (of the C-terminus of the CH3 domain), or D231 to L476, respectively, for IgG₄, wherein the numbering is according to Kabat.

The antibody construct of the invention comprises in an N- to C-terminal order:
(a) the first domain;
(b) a peptide linker having the amino acid sequence of SEQ ID NOs: 689;
(c) the second domain;
(d) a peptide linker having the amino acid sequence of SEQ ID NO: 694;
(e) the first polypeptide monomer of the third domain (comprising a hinge, a CH2 domain and a CH3 domain);
(f) a peptide linker having the amino acid sequence of SEQ ID NO: 697; and
(g) the second polypeptide monomer of the third domain (comprising a hinge, a CH2 domain and a CH3 domain).

The antibody construct of the invention comprises in an N- to C-terminal order:
(a) the first domain having the amino acid sequence of SEQ ID No: 179;
(b) a peptide linker having the amino acid sequence of SEQ ID NO: 689;
(c) the second domain having the amino acid sequence of SEQ ID NO: 642;
(d) a peptide linker having the amino acid sequence of SEQ ID NOs: 686; and
(e) the third domain having the amino acid sequence of SEQ ID NO: 700.

Hence, the antibody construct of the present invention comprises in an N- to C-terminal order:
(a) a polypeptide having the amino acid sequence of SEQ ID NO: 180;
(b) a linker having an amino acid sequence as depicted in SEQ ID NO: 686; and
(c) the third domain having an amino acid sequence as depicted in SEQ ID NO: 700.

The antibody construct of the present invention comprises or consists of a polypeptide having the amino acid sequence as depicted in SEQ ID NO: 661.

The antibody construct of the present invention, or one or both of its binding domains, may be "humanized" or "human". "Humanized" antibodies, variants or fragments thereof, antibody constructs and binding domains are based on immunoglobulins of mostly human sequences, which contain (a) minimal sequence(s) derived from non-human immunoglobulin. For the most part, humanized antibodies, variants or fragments thereof, antibody constructs and binding domains are based on human immunoglobulins (recipient antibodies) in which residues from a hypervariable region or CDR are replaced by residues from a hypervariable region or CDR of a non-human species (donor antibody) such as a rodent (e.g. mouse, hamster, rat or rabbit) having the desired specificity, affinity, capacity and/or biological activity. In some instances, Fv framework region (FR) residues of the human immunoglobulin are replaced by corresponding non-human residues. Furthermore, "humanized" antibodies, variants or fragments thereof, antibody constructs and binding domains as used herein may also comprise residues which are found neither in the recipient antibody nor the donor antibody. These modifications are made to further refine and optimize antibody performance. The humanized antibodies, variants or fragments thereof, antibody constructs and binding domains may also comprise at least a portion of an immunoglobulin constant region (such as Fc), typically that of a human immunoglobulin. For further details, see Jones et al., Nature, 321: 522-525 (1986); Reichmann et al., Nature, 332: 323-329 (1988); and Presta, Curr. Op. Struct. Biol., 2: 593-596 (1992).

Human anti-mouse antibody (HAMA) responses have led the industry to prepare chimeric or otherwise humanized antibodies / antibody constructs. It is however expected that certain human anti-chimeric antibody (HACA) responses will be observed, particularly in chronic or multi-dose utilizations of an antibody or antibody construct. Thus, it would be desirable to provide antibody constructs comprising a human binding domain against BCMA and/or a human binding domain against CD3, in order to vitiate concerns and/or effects of HAMA or HACA response.

Therefore, the first binding domain and the second binding domain are "human". The term "human antibody", "human antibody construct" and "human binding domain" includes antibodies, antibody constructs and binding domains, respectively, having antibody-derived regions such as variable and constant regions or domains which correspond substantially to human germline immunoglobulin sequences known in the art, including, for example, those described by Kabat *et al.* (1991) (*loc. cit*.)*.* The human antibody constructs or binding domains may include amino acid residues not encoded by human germline immunoglobulin sequences (*e.g.*, mutations introduced by random or site-specific mutagenesis *in vitro* or by somatic mutation *in vivo*)*,* for example in the CDRs, and in particular in CDR3. The human antibody constructs or binding domains can have at least one, two, three, four, five, or more positions replaced with an amino acid residue that is not encoded by the human germline immunoglobulin sequence. The definition of human antibodies, antibody constructs and binding domains as used herein also contemplates fully human antibodies, antibody constructs and binding domains which include only non-artificially and/or genetically altered human sequences of antibodies as those can be derived by using technologies or systems such as the Xenomouse.

Antibody constructs comprising at least one human binding domain avoid some of the problems associated with antibodies or antibody constructs that possess non-human such as rodent (*e.g.* murine, rat, hamster or rabbit) variable and/or constant regions. The presence of such rodent derived proteins can lead to the rapid clearance of the antibodies or antibody constructs or can lead to the generation of an immune response against the antibody or antibody construct by a patient. To avoid the use of rodent-derived antibody constructs, humanized or fully human antibody constructs can be generated through the introduction of human antibody function into a rodent so that the rodent produces fully human antibodies.

In some embodiments, the antibody construct of the invention is an "isolated" or "substantially pure" antibody construct. "Isolated" or "substantially pure", when used to describe the antibody constructs disclosed herein, means an antibody construct that has been identified, separated and/or recovered from a component of its production environment. Preferably, the antibody construct is free or substantially free of association with all other components from its production environment. Contaminant components of its production environment, such as that resulting from recombinant transfected cells, are materials that could interfere with diagnostic or therapeutic uses for the antibody construct, and may include enzymes, hormones, and other proteinaceous or non-proteinaceous compounds. It is understood that the isolated or substantially pure antibody construct may constitute from 5% to 99.9% by weight of the total protein / polypeptide content in a given sample, depending on the circumstances. The desired antibody construct may be produced at a significantly higher concentration through the use of an inducible promoter or high expression promoter. The definition includes the production of an antibody construct in a wide variety of organisms and/or host cells that are known in the art. In certain embodiments, the antibody construct will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver staining. Usually, however, an isolated antibody construct will be prepared by at least one purification step.

Peptides are short chains of amino acid monomers linked by covalent peptide (amide) bonds. Hence, peptides fall under the broad chemical classes of biological oligomers and polymers. Amino acids that are part of a peptide or polypeptide chain are termed "residues" and can be consecutively numbered. All peptides except cyclic peptides have an N-terminal residue at one end and a C-terminal residue at the other end of the peptide. An oligopeptide consists of only a few amino acids (usually between two and twenty). A polypeptide is a longer, continuous, and unbranched peptide chain. Peptides are distinguished from proteins on the basis of size, and as an arbitrary benchmark can be understood to contain approximately 50 or fewer amino acids. Proteins consist of one or more polypeptides, usually arranged in a biologically functional way. While aspects of the lab techniques applied to peptides versus polypeptides and proteins differ (e.g., the specifics of electrophoresis, chromatography, etc.), the size boundaries that distinguish peptides from polypeptides and proteins are not absolute. Therefore, in the context of the present invention, the terms "peptide", "polypeptide" and "protein" may be used interchangeably, and the term "polypeptide" is often preferred.

Polypeptides may further form multimers such as dimers, trimers and higher oligomers, which consist of more than one polypeptide molecule. Polypeptide molecules forming such dimers, trimers etc. may be identical or non-identical. The corresponding structures of higher order of such multimers are, consequently, termed homo- or heterodimers, homo- or heterotrimers etc. An example for a hereteromultimer is an antibody or immunoglobulin molecule, which, in its naturally occurring form, consists of two identical light polypeptide chains and two identical heavy polypeptide chains. The terms "peptide", "polypeptide" and "protein" also refer to naturally modified peptides / polypeptides / proteins wherein the modification is accomplished e.g. by post-translational modifications like glycosylation, acetylation, phosphorylation and the like. A "peptide", "polypeptide" or "protein" when referred to herein may also be chemically modified such as pegylated. Such modifications are well known in the art.

The antibody construct of the present invention is typically formulated in a pharmaceutical composition or a formulation. Materials of a pharmaceutical composition are usually formulated in concentrations that are acceptable for the site of administration. Formulations and compositions thus may be designed in accordance with the invention for delivery by any suitable route of administration.

As used herein, the term "pharmaceutical composition" relates to a composition which is suitable for administration to a patient, preferably a human patient. The particularly preferred pharmaceutical composition of this invention comprises one or a plurality of the antibody construct(s) of the invention, usually in a therapeutically effective amount. The pharmaceutical composition may further comprise suitable formulations of one or more (pharmaceutically effective) carriers, stabilizers, excipients, diluents, solubilizers, surfactants, emulsifiers, preservatives and/or adjuvants. Acceptable constituents of the composition are typically nontoxic to recipients at the dosages and concentrations employed. Pharmaceutical compositions of the invention include, but are not limited to, liquid, frozen, and lyophilized compositions. If the pharmaceutical composition has been lyophilized, the lyophilized material is reconstituted in an appropriate liquid prior to administration. The lyophilized material may e.g. be reconstituted in bacteriostatic water for injection (BWFI), physiological saline, phosphate buffered saline (PBS), or the same formulation the protein had been in prior to lyophilization.

The compositions may comprise a pharmaceutically acceptable carrier. In general, as used herein, "pharmaceutically acceptable carrier" means any and all aqueous and non-aqueous solutions, sterile solutions, solvents, buffers, e.g. phosphate buffered saline (PBS) solutions, water, suspensions, emulsions, such as oil/water emulsions, various types of wetting agents, liposomes, dispersion media and coatings, which are compatible with pharmaceutical administration, in particular with parenteral administration. The use of such media and agents in pharmaceutical compositions is well known in the art, and the compositions comprising such carriers can be formulated by well-known conventional methods.

Certain embodiments provide pharmaceutical compositions comprising the antibody construct of the invention and further one or more excipients. Excipients can be used for a wide variety of purposes, such as adjusting physical, chemical, or biological properties of formulations, such as adjustment of viscosity, and or processes of the invention to improve effectiveness and/or to stabilize such formulations and processes against degradation and spoilage e.g. due to stresses that occur during manufacturing, shipping, storage, pre-use preparation, administration, and thereafter. Excipients should in general be used in their lowest effective concentrations.

In certain embodiments, the pharmaceutical composition may also contain formulation materials / substances for the purpose of modifying, maintaining or preserving certain characteristics of the composition such as the pH, osmolarity, viscosity, clarity, color, isotonicity, odor, sterility, stability, rate of dissolution or release, adsorption or penetration (see, Remington's Pharmaceutical Sciences, 18" Edition, 1990, Mack Publishing Company).

As used herein, the singular forms "a", "an", and "the" include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within ±20%, preferably within ±15%, more preferably within ±10%, and most preferably within ±5% of a given value or range. It also includes the concrete value, *e.g.,* "about 50" includes the value "50".

Throughout this specification and the claims, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

A better understanding of the present invention and of its advantages will be obtained from the following examples, offered for illustrative purposes only. The examples are not intended and should not be construed as to limit the scope of the present invention in any way.

### EXAMPLES

Background: AMG 701, an HLE BiTE^{®} molecule which binds BCMA on Multiple Myeloma (MM) cells and CD3 on T cells, has activity in MM preclinical models. Objectives of this first-in-human study include evaluating safety and estimating the maximum tolerated dose (MTD) of AMG 701 in patients with relapsed/refractory (R/R) Multiple Myeloma (NCT03287908).

Methods: Weekly short-term IV infusions of AMG 701 were administered in 4-week cycles for as long as patients benefitted, first to single-patient cohorts (5, 15, 45 µg), then cohorts of ≥3 patients (140, 400, 800, 1200 µg), and possibly up to 10 patients in higher-dose cohorts (1600, 3000, 4500, 6500, 9000, 12000 µg). Eligible patients had MM relapsed after or intolerant to ≥2 lines with a proteasome inhibitor, IMiD, and anti-CD38 MAb (where approved and available). Excluded were patients with solely extramedullary disease (allowed in subsequent dose expansion), CNS involvement, prior BCMA-directed therapy, and immunosuppressive therapy. After a grade 3 dose-limiting toxicity (DLT) of cytokine release syndrome (CRS) at 1600 µg, for all target doses ≥1200 µg, an initial run-in dose of 800 µg was added, followed by step up to the target dose. MRD was defined as <1/105 cells, as measured per the FDA-authorized next generation sequencing assay

Results (not including the follow-up results shown in Table 2): 47 patients received AMG 701 at doses ranging from 5 to 6500 µg, with an 800 µg run-in dose used for step-up to target doses of≥1200 µg; patients are yet to be enrolled at additional planned doses of 9000 and 12000 µg, see also Figure 1. Median age was 62 years, 21 patients (78%) had ≥5 prior lines of therapy. 12 patients are ongoing, MTD as defined per protocol has not yet been reached. No anti-AMG 701 antibodies were detected. Responses included 1 stringent complete response (sCR) at 800 µg and 4 partial responses (PRs), 2 PRs at 3000 µg, 1 PR at 4500 µg, and 1 PR and a minimal response at 6500 µg. The patient with the sCR had an initial PR at 1 month, deepening to sCR at 4.3 months; this ongoing response has lasted 13.2 months, 9.8 of which were MRD-negative. The 4 PRs started at 1-3.7 months (responses were first assessed at 1 month), and the responses lasted up to 4 months (three ongoing).

Conclusions: In this FIH study with ongoing dose escalation and MTD not yet identified, AMG 701, an HLE antibody construct targeting CD3 and BCMA, showed encouraging activity in patients with heavily pre-treated R/R MM.

**Table 1: Results of the AMG 701 FIH study. The table does not include the single-patient cohorts 1-3 of 5, 15, and 45 µg. + indicates responses are ongoing. * Save for 1 patient at 1600 µg, i.e. the patient with the DLT of CRS, all patients receiving doses of ≥1200 µg received a step dose of 800 µg prior to receiving the target dose on Day 5 or 8. The # of patients receiving the target dose on Day 5 is as follows: 1600 µg (n=3), 3000 µg (n=2), and 4500 µg (n=2); all other patients received the target dose on Day 8.**

| | # of patients | Best | Any response (months) | |
|---|---|---|---|---|
| Cohort: target dose (µg)* | at dose | response | Onset | Duration |
| 4: 140 | 3 | - | | |
| 5: 400 | 4 | - | | |
| 6: 800 | 4 | sCR | 1.0 | 13.2+ |
| 7: 1600 | 1 | - | | |
| 8: 1200 | 4 | - | | |
| 9: 1600 | 8 | - | | |
| 10: 3000 | 10 | PR | 3.7 | 3.7+ |
| | | PR | 1.8 | 4.0 |
| 11: 4500 | 6 | PR | 1.9 | 1.0+ |
| 12: 6500 | 3 | PR | 1.0 (pend PR) | NA (pend PR) |
| | | MR | | |
| 13: 9000 | 3-10 | - | | |
| 14: 12000 | 4-6 | - | | |

**Table 2: Follow-up of the results of the AMG 701 FIH study. The table does not include the single-patient cohorts 1-3 of 5, 15, and 45 µg, and the table does not include cohorts 4-9 of 140 µg to 1600 µg (see Table 1). The sCR responder of cohort 6 (800 µg) in Table 1 has a response duration of 19.6+ months. + indicates responses are ongoing. All patients shown in this Table 2 received a step dose of 800 µg prior to receiving the target dose on day 3, 5 or 8.**

| Cohort | Target dose (µg) | Day to target dose | # of patients at dose | Best response | Any response (months) | |
|---|---|---|---|---|---|---|
| | | | | | Onset | Duration |
| 10 | 3000 | 8 | 11 | PR | 1.8 | 3.8 |
| | | 8 | | PR | 2.8 | 7.4 |
| | | 5 | | sCR | 1.0 | 3.9+ |
| 11 | 4500 | 8 | 7 | VGPR | 2.8 | 6.5+ |
| | | 5 | | VGPR | 1.9 | 4.7+ |
| 12 | 6500 | 8 | 9 | PR | 1.0 | 2.8+ |
| | | 8 | | VGPR | 1.9 | 5.8+ |
| | | 3 | | CR | 1.0 | 1.9+ |
| | | 3 | | VGPR | 1.0 | 6.5+ |

The IMWG response criteria for a complete response (see also http://imwg.myeloma.org/international-myeloma-working-group-imwg-uniform-response-criteria-for-multiple-myeloma/) are:
- Negative M protein immunofixation on the serum and urine,
- Disappearance of any soft tissue plasmacytomas, and
- < 5% plasma cells in bone marrow

The IMWG response criteria for a partial response are:
- M protein electrophoresis: ≥ 50% reduction of serum M-protein and reduction in 24 hours urinary M-protein by ≥90% or to <200 mg/24 h
- Free light chains (FLC): If the serum and urine M protein are unmeasurable, a ≥ 50% decrease in the difference between involved and uninvolved FLC levels is required in place of the M-protein criteria
- If serum and urine M protein are not measurable, and serum free light assay is also not measurable: ≥ 50% reduction in plasma cells is required in place of M protein, provided baseline bone marrow plasma cell percentage was ≥ 30%
- In addition to the above listed criteria, if present at baseline, a ≥ 50% reduction in the size of soft tissue plasmacytomas is also required

The IMWG response criteria for a very good partial response are:
- Serum and urine M protein detectable by immunofixation but not on electrophoresis or ≥ 90% reduction in serum M-protein plus urine M-protein level < 100 mg/24 h

## Claims

1. An antibody construct comprising the amino acid sequence as depicted in SEQ ID NO: 661, for use in the treatment or amelioration of a BCMA positive neoplasm in a human subject, wherein the BCMA positive neoplasm is relapsed and/or refractory multiple myeloma, wherein the antibody construct is administered at a minimum dose of 800 µg/day in at least one cycle, wherein one cycle comprises at least three individual administrations of the antibody construct, wherein the antibody construct is administered in one or two dose steps during the first cycle, wherein a first dose is between 800 µg/day and 1200 µg/day, and a last dose (target dose) is between 6500 µg/day and 12 mg/day, and
wherein the antibody construct is administered intravenously.

2. The antibody construct for use according to claim 1, wherein the antibody construct is administered for 2, 3, 4, 5, 6, 7, 8, 9, 10, 11, 12, 13, 14, 15, 16, 17, 18, 19, 20, 21, 22, 23, 24, 25 or more cycles, preferably wherein the antibody construct is administered during the second cycle and optionally during any subsequent cycle at constant doses.

3. The antibody construct for use according to any one of the preceding claims, wherein one cycle has about 25 to about 30 days, preferably about 26 or 27 to about 29 days, and more preferably about 28 days.

4. The antibody construct for use according to any one of the preceding claims, wherein the first cycle comprises or consists of three to six individual administrations, preferably four or five individual administrations of the antibody construct.

5. The antibody construct for use according to any one of the preceding claims, wherein the antibody construct is administered via intravenous bolus injection, bolus infusion or short-term intravenous infusion.

6. The antibody construct for use according to any one of the preceding claims, wherein the antibody construct has an elimination half-life (T_{1/2}) of about 3 to about 14 days, about 4 to about 12 days, about 3 or 4 to about 10 days, about 3 or 4 to about 8 days, or about 5 to about 7 days.

## Patentansprüche

1. Antikörperkonstrukt, umfassend die Aminosäuresequenz, wie sie unter SEQ ID NO: 661 dargestellt wird, zur Verwendung bei der Behandlung oder Verbesserung einer BCMA-positiven Neoplasie bei einem menschlichen Individuum, wobei es sich bei der BCMA-positiven Neoplasie um rezidiviertes und/oder refraktäres multiples Myelom handelt, wobei das Antikörperkonstrukt in einer Mindestdosis von 800 µg/Tag in mindestens einem Zyklus verabreicht wird, wobei ein Zyklus mindestens drei individuelle Verabreichungen des Antikörperkonstrukts umfasst, wobei das Antikörperkonstrukt in einem oder zwei Dosisschritten während des ersten Zyklus verabreicht wird, wobei eine erste Dosis zwischen 800 µg/Tag und 1200 µg/Tag liegt und eine letzte Dosis (Zieldosis) zwischen 6500 µg/Tag und 12 mg/Tag liegt, und
wobei das Antikörperkonstrukt intravenös verabreicht wird.

2. Antikörperkonstrukt zur Verwendung nach Anspruch 1, wobei das Antikörperkonstrukt für 2, 3, 4, 5, 6, 7, 8, 9, 10, 11, 12, 13, 14, 15, 16, 17, 18, 19, 20, 21, 22, 23, 24, 25 oder mehr Zyklen verabreicht wird, vorzugsweise wobei das Antikörperkonstrukt während des zweiten Zyklus und gegebenenfalls während eines nachfolgenden Zyklus in konstanten Dosen verabreicht wird.

3. Antikörperkonstrukt zur Verwendung nach einem der vorhergehenden Ansprüche, wobei ein Zyklus etwa 25 bis etwa 30 Tage, vorzugsweise etwa 26 oder 27 bis etwa 29 Tage und weiter bevorzugt etwa 28 Tage aufzeigt.

4. Antikörperkonstrukt zur Verwendung nach einem der vorhergehenden Ansprüche, wobei der erste Zyklus drei bis sechs individuelle Verabreichungen, vorzugsweise vier oder fünf individuelle Verabreichungen des Antikörperkonstrukts, umfasst oder daraus besteht.

5. Antikörperkonstrukt zur Verwendung nach einem der vorhergehenden Ansprüche, wobei das Antikörperkonstrukt durch intravenöse Bolusinjektion, Bolusinfusion oder kurzzeitige intravenöse Infusion verabreicht wird.

6. Antikörperkonstrukt zur Verwendung nach einem der vorhergehenden Ansprüche, wobei das Antikörperkonstrukt eine Eliminationshalbwertszeit (T_{1/2}) von etwa 3 bis etwa 14 Tagen, etwa 4 bis etwa 12 Tagen, etwa 3 oder 4 bis etwa 10 Tagen, etwa 3 oder 4 bis etwa 8 Tagen oder etwa 5 bis etwa 7 Tagen aufweist.

## Revendications

1. Construction d'anticorps comprenant la séquence d'acides aminés telle que décrite dans SEQ ID NO: 661, pour utilisation dans le traitement ou l'amélioration d'un néoplasme positif pour BCMA chez un sujet humain, dans laquelle le néoplasme positif pour BCMA est un myélome multiple récurrent et/ou réfractaire, la construction d'anticorps étant administrée à une dose minimale de 800 µg/jour en au moins un cycle, un cycle comprenant au moins trois administrations individuelles de la construction d'anticorps, la construction d'anticorps étant administrée en une ou deux étapes de dose au cours du premier cycle, une première dose étant comprise entre 800 µg/jour et 1 200 ug/jour, et une dernière dose (dose cible) étant comprise entre 6 500 µg/jour et 12 mg/jour, et
la construction d'anticorps étant administrée par voie intraveineuse.

2. Construction d'anticorps pour utilisation selon la revendication 1, la construction d'anticorps étant administrée pendant 2, 3, 4, 5, 6, 7, 8, 9, 10, 11, 12, 13, 14, 15, 16, 17, 18, 19, 20, 21, 22, 23, 24, 25 cycles ou plus, de préférence la construction d'anticorps étant administrée pendant le deuxième cycle et facultativement pendant un cycle subséquent quelconque à des doses constantes.

3. Construction d'anticorps pour utilisation selon l'une quelconque des revendications précédentes, un cycle ayant environ 25 à environ 30 jours, de préférence environ 26 ou 27 à environ 29 jours, et plus préférablement environ 28 jours.

4. Construction d'anticorps destinée à être utilisée selon l'une quelconque des revendications précédentes, dans laquelle le premier cycle comprend ou est constitué de trois à six administrations individuelles, de préférence quatre ou cinq administrations individuelles de la construction d'anticorps.

5. Construction d'anticorps pour utilisation selon l'une quelconque des revendications précédentes, la construction d'anticorps étant administrée par injection de bolus intraveineuse, perfusion de bolus ou perfusion intraveineuse de courte durée.

6. Construction d'anticorps pour utilisation selon l'une quelconque des revendications précédentes, la construction d'anticorps ayant une demi-vie d'élimination (T_{1/2}) d'environ 3 à environ 14 jours, d'environ 4 à environ 12 jours, d'environ 3 ou 4 à environ 10 jours, d'environ 3 ou 4 à environ 8 jours, ou d'environ 5 à environ 7 jours.
